# EUROPEAN PATENT APPLICATION

(11) **EP 4 012 637 A1**
(43) Date of publication of application: **15.06.2022**
(21) Application number: 20853149.1
(22) Date of filing: 21.01.2020
(51) Int. Cl.: G06Q 20/06, G06Q 50/10, G16H 20/30

(54) **PROGRAM, CHALLENGE ASSISTANCE SYSTEM, CHALLENGE ASSISTANCE METHOD, AND TERMINAL**

(30) Priority: 09.08.2019 JP 2019147695
(71) Applicant: Tanimoto, Hiroshi, Tokyo 102-0083 (JP)
(72) Inventor: Tanimoto, Hiroshi, Tokyo 102-0083 (JP)
(74) Representative: FRKelly
(86) International application number: PCT/JP2020/001999
(87) International publication number: WO 2021/029090

(57) **Abstract**

The purpose of the present invention is to provide a program that can realize a challenge assistance system to increase the possibility that a user will achieve a challenge. A program causing a terminal connected to the Internet (50) to execute: in a blockchain network (55) having a first smart contract which determines whether or not contract terms related to a challenge to be achieved by a first user are met to allocate a first token based on the determination result of the contract terms, and a second smart contract which generates a transaction to allocate the first token to a second user based on the balance of a second token of the second user, a process of acquiring determination data; a process of transmitting the determination data to the blockchain network (55); a process of generating a transaction used to allocate the second token; and a process of transmitting, to the blockchain network (55), the transaction used to allocate the second token.

## Description

### Technical Field

The present invention relates to a program, a challenge assistance system, a challenge assistance method, and a terminal.

### Background Art

Everybody sometimes has challenges which they want to achieve but it is hard to realize. The challenges include, for example, walking to maintain and improve health, going to a medical checkup, quitting smoking, studying English conversation, and the like.

In the case of a challenge of this type, someone other than a person who does the challenge is often expecting the person to achieve the challenge for the health of the person or skill acquisition. For example, the father of a person living an unhealthy life hopes to let the person do healthy activities such as walking somehow, or the wife and child of the person want to support the efforts of the person for healthy activities while hoping that the person will be healthy for a long time.

### Summary

### Technical Problem

The person who does a challenge of this type needs to motivate himself properly to do the challenge. Further, the fact that the challenge is not achieved is undesirable not only for the person but also for other people who expect the person to achieve the challenge. Other people who expect the person to achieve the challenge can motivate the person to do the challenge.

However, it is difficult for other people who expect the person to achieve the challenge to continue to support the person actively in such a situation that other people do not have any benefits that immediately appear in addition to the benefit that the person achieves the challenge.

When maintaining/increasing of motivation by the person himself or maintaining/increasing of motivation of the person with the support of other people is insufficient, the person becomes reluctant to achieve the challenge, and the possibility of not achieving the challenge increases.

Therefore, it is an object of the present invention to provide a program that can realize a challenge assistance system to increase the possibility that a user will achieve a challenge.

### Solution to Problem

A program according to one aspect of the present invention causes a terminal connected to the Internet to execute: in a blockchain network connected to the Internet and having a first smart contract which determines whether or not contract terms related to a challenge including conditions to achieve the challenge to be achieved by a first user and a deadline to achieve the challenge are met, and generates a transaction to allocate a first token based on the determination result of the contract terms, and a second smart contract to which the first token is allocated from the first smart contract, and which manages the balance of a second token allocatable from the first user to a second user and generates a transaction to allocate the first token to the second user based on the balance of the second token of the second user, a process of acquiring determination data used for the determination of the contract terms; a process of transmitting the determination data to the first smart contract; a process of generating a transaction used to allocate the second token from the first user to the second user by the second smart contract; and a process of transmitting, to the second smart contract, the transaction used to allocate the second token from the first user to the second user.

According to this aspect, the first token to be allocated by the first smart contract when the challenge is achieved can be allocated to the second user based on the balance of the second token allocated by the first user. The first user can allocate the second token to the second user before the start of the challenge, in the process of achieving the challenge, or before and after the achievement of the challenge.

The second user can get the allocation of the first token by holding the second token. Thus, the second user is motivated to support for the challenge of the first user actively to earn the first token in order to get the second token from the first user in compensation for the support. The first user gets motivated for the challenge by being supported by the second user. Since a situation of involving the second user in achieving the challenge, not by the first user alone, is created, the possibility that the first user will achieve the challenge increases.

In the above aspect, the program may also cause the terminal to execute: a process of generating token issuance information used to issue the second token; and a process of transmitting the token issuance information to the blockchain network.

According to this aspect, the first user can operate the terminal to issue the second token. The percentage of allocation to the second user can be adjusted properly by allowing the first user to issue the second token freely. The percentage is so adjusted that a user who makes a higher contribution to support the first user gets more allocation of the second token. Therefore, it will be easier for the first user to appeal the second user and other users to support the first user. Thus, the first user gets more support to increase the possibility that the first user will achieve the challenge.

In the above aspect, the program may also cause the terminal to execute: a process of generating a transaction used to allocate the second token from the second user to a third user; and a process of transmitting, to the second smart contract, the transaction used to allocate the second token from the second user to the third user.

According to this aspect, the second user can send the second token to the third user. The second token can circulate from one user to another. The second token is allocated to a user different from the user selected by the first user. Therefore, the first user can get support from more users, and the possibility that the first user will achieve the challenge increases.

In the above aspect, the program may also cause the terminal to execute: in the blockchain network further having a third smart contract which manages a balance of a third token, a process of generating a transaction used to allocate the third token by the third smart contract based on the balance of the second token; and a process of transmitting, to the third smart contract, the transaction used to allocate the third token based on the balance of the second token.

According to this aspect, the balance of the third token is managed based on the balance of the second token. The first user can get long-term support by setting the second token as a temporary token for support of each challenge of the first user, and the third token as a long-term token for two or more challenges of the first user. The first user having a sense of security to receive long-term support gets motivated for the challenges stably. Therefore, the possibility that the first user will achieve the challenges increases.

In the above aspect, the program may also cause the terminal to execute: a process of generating a transaction used to allocate the third token by the third smart contract based on the balance of the second token and a difficulty level of the challenge; and a process of transmitting, to the third smart contract, the transaction used to allocate the third token based on the balance of the second token and the difficulty level of the challenge.

By allocating the third token based on the difficulty level of the challenge, the third token can be allocated properly according to the difficulty level of a third challenge, rather than to allocate the third token uniformly in each challenge from the balance of the second token.

In the above aspect, the program may also cause the terminal to execute: a process of generating information indicative of an termination of the challenge; and a process of transmitting, to the first smart contract, the information indicative of the termination of the challenge to cause the first smart contract to generate a transaction used to allocate the first token according to an allocation setting of the first token when the challenge is terminated.

According to this aspect, the challenge can be terminated, and this increases convenience as a system.

In the above aspect, the program may also cause the terminal to execute: a process of acquiring a transmission destination to transmit information about the challenge of the first user; and a process of transmitting, to the transmission destination, the information about the challenge of the first user to generate a transaction used to allocate a prescribed token in the blockchain network from a prescribed user of the blockchain network to the first user.

According to this aspect, the prescribed user of the blockchain network can allocate the prescribed token to the first user. Based on information about the challenge of the first user, the first user can receive the token from the prescribed user and get support from the prescribed user. Therefore, the possibility that the first user will achieve the challenge increases.

A program according to another aspect of the present invention causes a terminal connected to the Internet to execute: a process of generating contract terms related to a challenge including conditions to achieve the challenge to be achieved by a first user and a deadline to achieve the challenge, and contract information including an amount of first token deposited for the challenge, an allocation setting of the first token when the challenge is successful, and an allocation setting of the first token when the challenge is unsuccessful; and a process of determining whether or not the contract terms are met based on the contract terms and determination data used for the determination of the contract terms, and transmitting, to a blockchain network, a contract generation transaction based on the contract information to generate, in the blockchain network connected to the Internet, a first smart contract which generates a transaction to allocate the first token based on the determination result of the contract terms.

According to this aspect, the first smart contract to achieve the challenge can be generated from the terminal connected to the Internet. Since any terminal connected to the Internet can generate the first smart contract related to the challenge freely, a challenge in line with the purpose of each user can be achieved. The motivation of the user for the challenge set in line with the purpose of the user increases, and the possibility that the user will achieve the challenge increases.

In the above aspect, the program may also cause the terminal to execute: information indicative of an termination of the challenge; and a process of transmitting, to the first smart contract, the information indicative of the termination of the challenge to cause the first smart contract to generate a transaction to allocate the first token according to an allocation setting of the first token when the challenge is terminated.

This can cause a creator of the first smart contract related to the challenge to terminate the challenge, and this increases convenience as a system.

In the above aspect, the first smart contract may also calculate an achievement level of the challenge based on the contract terms and the determination data, and generate a transaction to allocate the first token based on the calculated achievement level.

According to this aspect, the first token is allocated based on the achievement level even when the challenge is terminated or unsuccessful. When the difficulty level of the challenge is set higher, the possibility that the challenge will be terminated or unsuccessful may increase. In this case, since the first token is allocated even when the challenge is terminated, the user can stay motivated for the challenge to promote the achievement of the challenge.

In the above aspect, the determination data may also include end determination data to determine the end of the challenge.

According to this aspect, the first smart contract can acquire information (oracle) from the outside to determine the end of the challenge to end the challenge without fail.

In the above aspect, the end determination data may also include data about the date and time.

According to this aspect, when the first smart contract cannot automatically acquire the information about the date and time from the Internet or the blockchain network, the information about the date and time can be transmit to the first smart contract. Therefore, when the deadline of the challenge has passed, the challenge can be ended without fail.

A program according to still another aspect of the present invention causes a terminal connected to the Internet to execute: a process of determining whether or not contract terms related to a challenge including conditions to achieve the challenge to be achieved by a first user and a deadline to achieve the challenge are met, and generating a transaction to allocate a first token based on the determination result of the contract terms; and transmitting, to a blockchain network, a contract generation transaction to generate, in the blockchain network, a smart contract which manages a balance of a second token allocatable from the first user to a second user and activated after the determination is made, and generates a transaction to allocate the second token to the second user based on the determination result of the contract terms.

According to this aspect, the first user can allocate, to the second user, the second token activated after it is determined whether or not the contract terms related to the challenge are met. Therefore, the second user is motivated to give the first user support for the challenge in order to activate the second token. The first user gets motivated to do the challenge by being supported by the second user. Since a situation of involving the second user in achieving the challenge, not by the first user alone, is created, the possibility that the first user will achieve the challenge increases.

A program according to yet another aspect of the present invention causes a terminal connected to the Internet to execute: in a blockchain network having a first smart contract which determines whether or not contract terms related to a challenge including conditions to achieve the challenge to be achieved by a prescribed user and a deadline to achieve the challenge are met based on the contract terms and determination data used for the determination of the contract terms, a second smart contract which manages a token balance in the blockchain network connected to the Internet based on the determination result transmitted from the first smart contract, and the second smart contract in which a token from the blockchain network is accumulated, and which allocates the accumulated token based on the determination result transmitted from the first smart contract, a process of generating a transaction to bet the token on a prediction of the determination result; and a process of transmitting, to the second smart contract, the transaction to bet the token on the prediction of the determination result.

According to this aspect, the second smart contract bets the token on the determination result of the challenge of the first user and manages the token balance based on the determination result to allocate the token. Therefore, more users have an interest in the first challenge in order to increase own tokens. When the own challenge attracts a lot of interest, the first user tries to achieve the challenge in response to the interest of the users. This increases the possibility that the first user will achieve the challenge.

A challenge assistance system according to yet another aspect of the present invention includes a first terminal and a second terminal connected to the Internet, wherein the first terminal includes: a contract information generation unit which generates contract terms related to a challenge including conditions to achieve the challenge to be achieved by a first user and a deadline to achieve the challenge, and contract information including the amount of first token deposited for the challenge, an allocation setting of the first token when the challenge is successful, and an allocation setting of the first token when the challenge is unsuccessful; and a first transaction generation unit which determines whether or not the contract terms are met based on the contract terms and determination data used for the determination of the contract terms, and transmits, to a blockchain network, a contract generation transaction based on the contract information to generate, in the blockchain network connected to the Internet, a smart contract which generates a transaction to allocate the first token based on the determination result of the contract terms, and the second terminal includes: a determination data acquisition unit which acquires the determination data used for the determination of the contract terms; and a second transaction generation unit which transmits the determination data to the smart contract.

According to this aspect, the smart contract is generated on the blockchain network by the contract information generated by the first terminal. The first token is deposited in the smart contract. The user gets motivated to do the challenge in order to get the first token allocated according to whether or not the contract terms related to the challenge are met. The determination data used for the determination of the contract terms is transmitted to the smart contract by the second terminal. The smart contract determines whether or not the contract terms are met according to the determination data.

The allocation of the first token according to the challenge result is reliably fulfilled even in the absence of an administrator by making the determination of the challenge through the smart contract. Therefore, the user can create a challenge promised to allocate a reward for the challenge result by generating the smart contract in the blockchain network. The challenge assistance system motivates the user to do the challenge by increasing the reliability of earning the reward for the challenge result to increase the possibility that the user will achieve the challenge.

### Advantageous Effect of Invention

According to the present invention, there can be provided a program that can realize a challenge assistance system to increase the possibility that a user will achieve a challenge.

### Brief Description of Drawings

Fig. 1 is a diagram illustrating the configuration of a challenge assistance system according to a first embodiment.
Fig. 2 is a block diagram of a fund provider terminal according to the first embodiment.
Fig. 3 is a block diagram of a challenger terminal according to the first embodiment.
Fig. 4 is a block diagram of a supporter terminal according to the first embodiment.

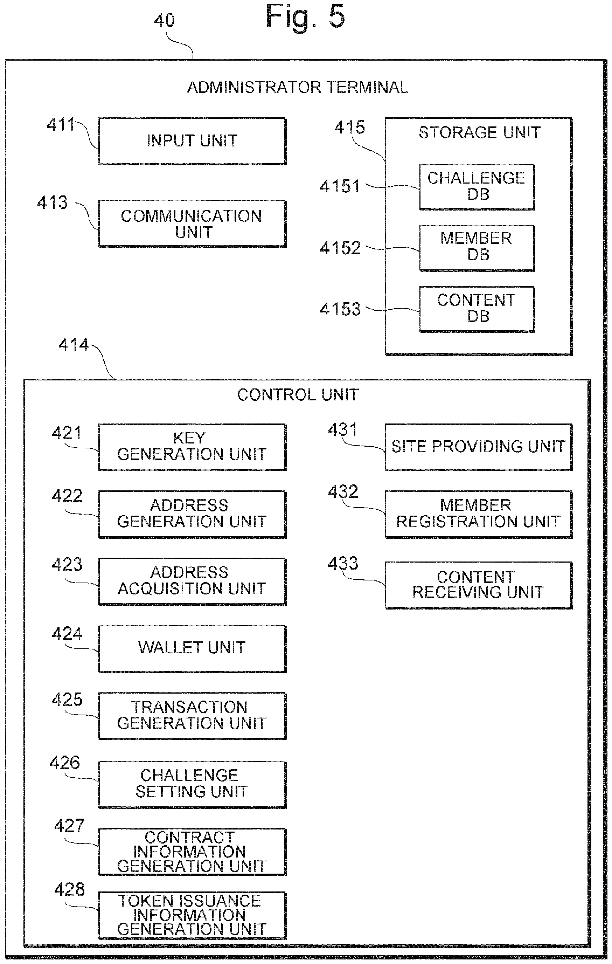

Fig. 5 is a block diagram of an administrator terminal according to the first embodiment.
Fig. 6 is a data structure stored in the administrator terminal according to the first embodiment.
Fig. 7 is a diagram illustrating smart contracts in a blockchain network according to the first embodiment.

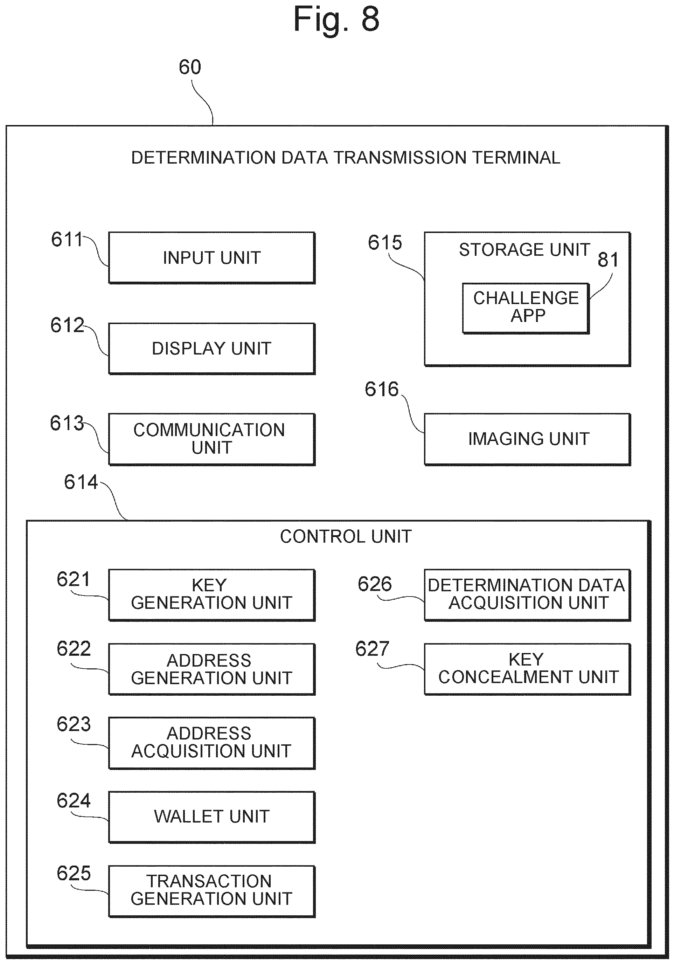

Fig. 8 is a block diagram of a determination data transmission terminal according to the first embodiment.
Fig. 9 is a sequence diagram of the challenge assistance system according to the first embodiment.
Fig. 10 is a sequence diagram of the challenge assistance system according to the first embodiment.
Fig. 11 is a diagram for describing a cheering token contract according to the first embodiment.
Fig. 12 is a diagram for describing the cheering token contract according to the first embodiment.
Fig. 13 is a diagram for describing a thanks token contract according to the first embodiment.
Fig. 14 is a diagram of a data structure stored in the fund provider terminal according to the first embodiment.
Fig. 15 is an example of contract information created by the fund provider terminal according to the first embodiment.
Fig. 16 is a diagram for describing a challenge contract according to the first embodiment.
Fig. 17 is a data structure stored in the administrator terminal according to the first embodiment.
Fig. 18 is a data structure stored in the administrator terminal according to the first embodiment.
Fig. 19 is a data structure of determination data according to the first embodiment.
Fig. 20 is a data structure of determination data recorded in the challenge contract according to the first embodiment.
Fig. 21 is a data structure stored in the challenger terminal according to the first embodiment.
Fig. 22 is a diagram for describing the thanks token contract according to the first embodiment.
Fig. 23 is an example of another challenge according to the first embodiment.
Fig. 24 is an example of still another challenge according to the first embodiment.
Fig. 25 is an example of yet another challenge according to the first embodiment.
Fig. 26 is an example of yet another challenge according to the first embodiment.
Fig. 27 is a screen displayed by a challenge app according to the first embodiment.
Fig. 28 is a screen displayed by the challenge app according to the first embodiment.
Fig. 29 is a screen displayed by the challenge app according to the first embodiment.
Fig. 30 is a screen displayed by the challenge app according to the first embodiment.
Fig. 31 is a screen displayed by the challenge app according to the first embodiment.
Fig. 32 is a screen displayed by the challenge app according to the first embodiment.
Fig. 33 is a screen displayed by the challenge app according to the first embodiment.
Fig. 34 is a diagram illustrating the configuration of a challenge assistance system according to a second embodiment.
Fig. 35 is a diagram illustrating smart contracts in a blockchain network according to a third embodiment.
Fig. 36 is a diagram for describing a thanks token contract according to the third embodiment.
Fig. 37 is a diagram for describing the thanks token contract according to the third embodiment.
Fig. 38 is a diagram for describing the thanks token contract according to the third embodiment.
Fig. 39 is a diagram for describing the thanks token contract according to the third embodiment.
Fig. 40 is a diagram illustrating smart contracts in a blockchain network according to a fourth embodiment.
Fig. 41 is a diagram for describing token management in the blockchain network according to the fourth embodiment.
Fig. 42 is a diagram for describing token management in the blockchain network according to the fourth embodiment.
Fig. 43 is a diagram for describing another example of token management in the blockchain network according to the fourth embodiment.
Fig. 44 is a diagram illustrating smart contracts in a blockchain network according to a fifth embodiment.

### Description of Embodiments

Preferred embodiments of the present invention will be described with reference to the accompanying drawings. Note that the same reference numerals in respective drawings have the same or similar components.

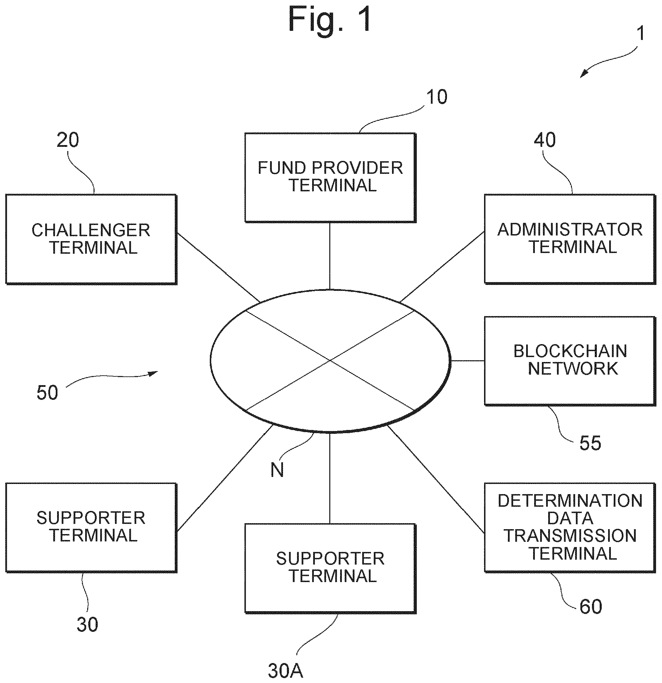

A first embodiment will be described. A challenge assistance system according to the first embodiment is illustrated in Fig. 1. A challenge assistance system 1 includes a fund provider terminal 10, a challenger terminal 20, supporter terminals 30, 30A, an administrator terminal 40, the Internet 50, a blockchain network 55, and a determination data transmission terminal 60.

The Internet 50 is so configured that the fund provider terminal 10, the challenger terminal 20, the supporter terminals 30, 30A, the administrator terminal 40, and the determination data transmission terminal 60 are connected through a network N. The blockchain network 55 is connected to the Internet 50.

The blockchain network 55 is a system in which a ledger with transactions recorded therein is shared and managed among multiple terminals. The blockchain is data in which multiple blocks are connected in a chain in chronological order, and each block includes transaction data in a certain period. In the blockchain technology, since multiple blocks are added in a state of retaining past information, the blockchain has the characteristic of being difficult to tamper the history.

In the present embodiment, it is assumed that Ethereum capable of realizing a smart contract is used as the blockchain network 55. However, any other blockchain network capable of realizing the smart contract may also be used.

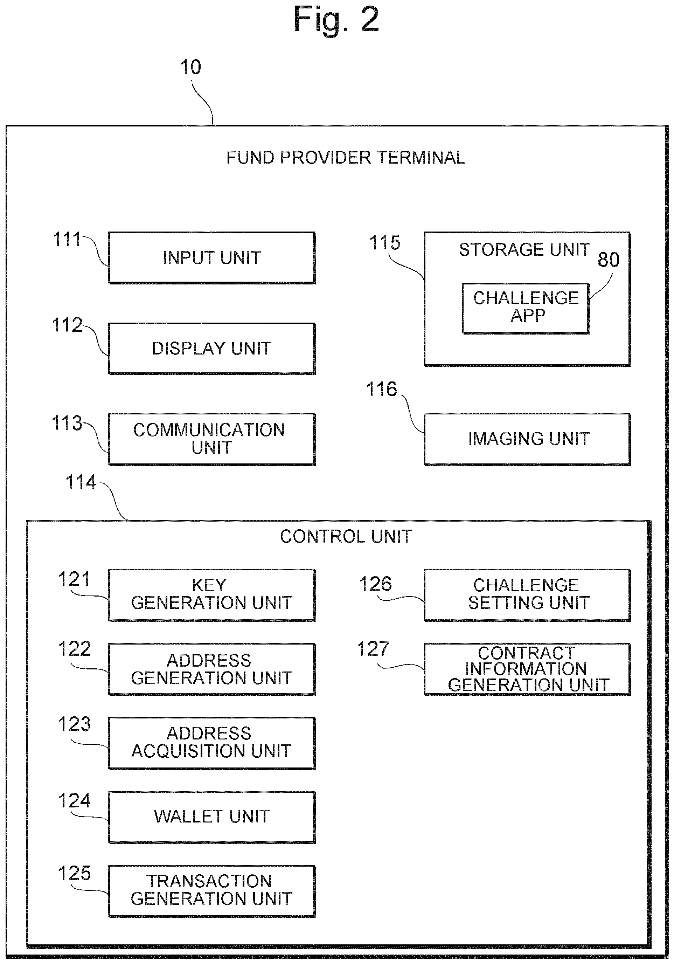

Fig. 2 illustrates a block diagram of the fund provider terminal 10. The fund provider terminal 10 is an information processing terminal having a function to perform communication with the administrator terminal 40, the Internet 50, and the blockchain network 55 through the network N. For example, the fund provider terminal 10 may be, but not limited to, a smartphone, a PC, a tablet, or the like.

The fund provider terminal 10 includes an input unit 111, a display unit 112, a communication unit 113, a control unit 114, a storage unit 115, and an imaging unit 116.

The input unit 111 accepts operations from a user. The display unit 112 provides a display of information to the user. The input unit 111 and the display unit 112 may also be united like a touch panel or the like. The communication unit 113 performs communication with external terminals through the network N. A challenge app 80 is stored in the storage unit 115. The imaging unit 116 is used to acquire images and the like.

Based on information input through the input unit 111, the user account and password of the challenge app 80 are stored in the storage unit 115. The fund provider terminal 10 transmits user identification information related to the stored user account to the administrator terminal 40.

The user identification information is information generated by using part or all of member information acquired by causing the user to enter, for example, at the time of installation or startup of the challenge app 80. The user identification information includes name, gender, date of birth, account name, public address of a determination data transmission terminal used by the user, and the like.

The control unit 114 includes a CPU and a memory. The control unit 114 functions as a key generation unit 121, an address generation unit 122, an address acquisition unit 123, a wallet unit 124, a transaction generation unit 125, a challenge setting unit 126, and a contract information generation unit 127 when the challenge app 80 (program) stored in the storage unit 115 is read into a RAM and executed by the CPU.

The key generation unit 121 generates a pair of a secret key and a public key unique to each user of the fund provider terminal 10. The generated secret key is stored in the storage unit 115. The secret key is used as an electronic signature upon transmission of a transaction to the blockchain network 55. It is desirable that the secret key be generated while offline and usually saved offline in a manner to be able to be connected as needed.

The address generation unit 122 generates a public address in the blockchain network 55 based on the public key generated by the key generation unit 121. The generated public address of a fund provider is stored in the storage unit 115. The public address is used to identify the user of the blockchain network 55. In the blockchain network 55, the sending destination of Ether as cryptocurrency in the Ethereum and any other token by a transaction is the public address. In the following, Ether is referred to as ETHER. The abbreviation of ETHER is ETH.

The address generation unit 122 transmits, to the administrator terminal 40 to be described later, the generated public address in association with the user identification information. By associating the public address with the user identification information, it can be confirmed that a public address in the blockchain network 55 is that of a user of the challenge assistance system 1.

The address acquisition unit 123 acquires any other public address in the blockchain network 55 from the administrator terminal 40 or any other terminal. When acquiring the other public address from the administrator terminal 40, the address acquisition unit 123 performs communication with the administrator terminal 40 through the network N to acquire the other public address stored in the administrator terminal 40.

When acquiring the other public address from the other terminal, for example, an image such as two-dimensional barcode indicative of the public address is read by using the imaging unit 116 to acquire the other public address. Alternatively, a public address represented by an image or text generated by the other terminal is acquired through the network N to acquire the other public address. The public address acquired by the address acquisition unit 123 is stored in the storage unit 115.

When there is user identification information in the public address acquired from the administrator terminal 40 or the other terminal, where the user identification information is generated by an app of the same type as the challenge app 80 stored in the other terminal, the address acquisition unit 123 can also acquire the user identification information. The acquired user identification information is stored in the storage unit 115.

The address acquisition unit 123 can acquire, from the administrator terminal 40 or the other terminal, the contract address of a smart contract related to a challenge in the blockchain network 55.

The wallet unit 124 has a function to manage a cryptocurrency balance. The cryptocurrency in the first embodiment is ETHER or a token uniquely issued in the blockchain network 55. The wallet unit 124 acquires the cryptocurrency balance from a history of transactions recorded in the blockchain network 55. The user can check the acquired balance by displaying the cryptocurrency balance on the display unit 112.

The wallet unit 124 generates fund payment data including a public address of a remittance destination or an account name corresponding to the public address of the remittance destination, and the amount of remittance. For example, the wallet unit 124 generates the fund payment data including the account specified by the user through the input unit 111 and the amount of remittance. Note that the words of remittance and funds are used in the sense of transfer of the cryptocurrency, and the transfer target is not limited to ETHER and any other token is also included.

The transaction generation unit 125 generates a transaction signed with the secret key stored in the storage unit 115, and transmits the transaction to the blockchain network 55. For example, the transaction generation unit 125 generates the transaction signed with the secret key stored in the storage unit 115 based on the fund payment data generated by the wallet unit 124, and transmits the transaction to the blockchain network 55. When the transaction is approved in the blockchain network 55, the transfer of funds based on the transaction is stored in the blockchain network 55.

The wallet unit 124 may acquire, from the administrator terminal 40, a list of challenge contracts to be described later, and provide a display to the user in a selectable format. Further, when acquiring the list of challenge contracts, the wallet unit 124 may also transmit a public address of the storage unit 115 so that the user can acquire, as a fund provider, a list of contract addresses of challenge contracts registered in the administrator terminal 40.

The challenge setting unit 126 acquires, from the user, settings for each challenge created by the fund provider to generate setting data. The setting data includes information such as the public address of the fund provider, the public address of each challenger who tries a challenge, the public address of an administrator, the amount of funds, a challenge start deadline, and a challenge period. Further, contract terms such as the specific content of a challenge to achieve the challenge, challenge achievement conditions, fund allocation settings when the challenge is successful, and fund allocation settings when the challenge is unsuccessful are included.

Based on the setting data generated by the challenge setting unit 126, the contract information generation unit 127 compiles contract/code written in Solidity language by sole to generate the compiled contract/code.

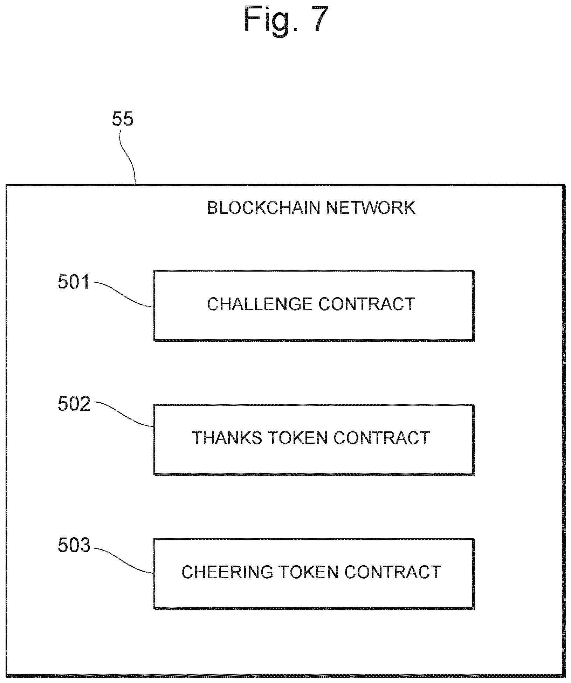

The transaction generation unit 125 transmits the complied contract/code generated by the contract information generation unit 127 to the blockchain network 55 as a transaction. When the transaction is approved in the blockchain network 55, a challenge contract 501 as a smart contract is generated in the blockchain network 55 as illustrated in Fig. 7. The challenge contract 501 will be described later. Note that the language used by the fund provider terminal 10 is not limited to the Solidity language, and any other language capable of generating a smart contract in the blockchain network 55 can be used.

The challenge contract 501 generated in the blockchain network 55 has a contract address in the blockchain network 55. A fund deposited by the fund provider is sent to the contract address of the challenge contract 501 and held. When the challenge contract 501 is generated, the contract address of the challenge contract 501 is transmitted and acquired from the blockchain network 55 to the fund provider terminal 10, or the fund provider terminal 10 acquires the contract address of the challenge contract 501 from the blockchain network 55. The acquired contract address of the challenge contract 501 is transmitted from the fund provider terminal 10 to the administrator terminal 40 together with the setting data generated by the challenge setting unit 126.

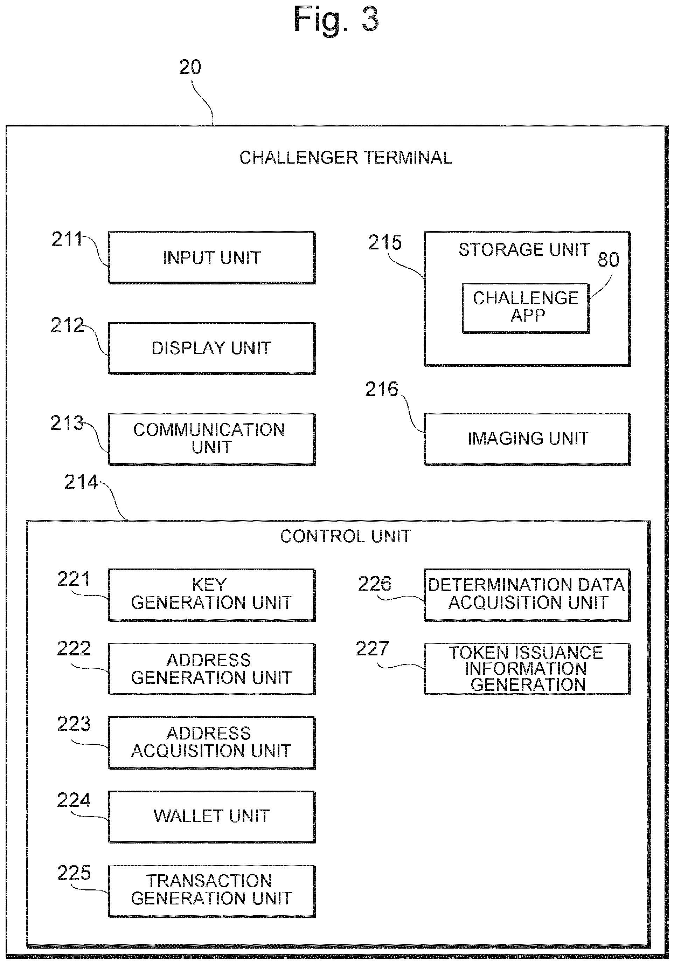

A block diagram of the challenger terminal 20 is illustrated in Fig. 3. The challenger terminal 20 is an information processing terminal having a function to perform communication with an administrator server and the blockchain network 55 through the network N. For example, the challenger terminal 20 may be, but not limited to, a smartphone, a PC, a tablet, or the like.

The challenger terminal 20 includes an input unit 211, a display unit 212, a communication unit 213, a control unit 214, a storage unit 215, and an imaging unit 216.

The input unit 211, the display unit 212, the communication unit 213, and the imaging unit 216 are the same as the input unit 111, the display unit 112, the communication unit 113, and the imaging unit 116. The challenger terminal 20 transmits own user identification information to the administrator terminal 40. The challenge app 80 is stored in the storage unit 215.

The control unit 214 includes a CPU and a memory. The control unit 214 functions as a key generation unit 221, an address generation unit 222, an address acquisition unit 223, a wallet unit 224, a transaction generation unit 225, a determination data acquisition unit 226, and a token issuance information generation unit 227 when the challenge app 80 (program) stored in the storage unit 215 is read into a RAM and executed by the CPU.

The key generation unit 221, the address generation unit 222, the address acquisition unit 223, the wallet unit 224, and the transaction generation unit 225 have the same functions as the key generation unit 121, the address generation unit 122, the address acquisition unit 123, the wallet unit 124, and the transaction generation unit 125.

A secret key generated by the key generation unit 221 is stored in the storage unit 215. The public address of a challenger generated by the address generation unit 222 is stored in the storage unit 215.

The determination data acquisition unit 226 acquires determination data used to determine whether or not contract terms are met from information input from the input unit 211 or information recorded in the storage unit 215 of the challenger terminal 20. As the information recorded in the storage unit 215, for example, there is exercise information such as the number of steps aggregated by a pedometer function of a smartphone and recorded in the storage unit 215. Alternatively, there is exercise information such as the number of steps recorded in the storage unit 215 from an external terminal capable of measuring the number of steps through the communication unit 213.

As the determination data, any data such as data as the evidence of activities to achieve a challenge and data including either "success" or "failure" of the challenge can be used.

The determination data can also include data indicative of an "termination" of the challenge. When the data indicative of the "termination" of the challenge is transmitted to the blockchain network 55, the challenge contract 501 can be set to perform a predetermined process.

The determination data includes end determination data to determine the success or failure of the challenge. The end determination data is, for example, the last day result of the challenge over multiple days. Further, the end determination data may also be information about the date and time for determining whether or not the deadline for the challenge has passed.

The transaction generation unit 225 generates a transaction signed with a secret key stored in the storage unit 215 to transmit the determination data to a smart contract. The transaction generation unit 225 transmits the transaction related to the determination data to the smart contract. When the transaction is approved in the blockchain network 55, data of the smart contract as the transaction destination is updated. The destination is, for example, the contract address of the challenge contract 501.

When the determination data acquired by the determination data acquisition unit 226 is transmitted to the smart contract, the transaction generation unit 225 may acquire, from the administrator terminal 40, the contract address of the smart contract, and provide a display to the user as the destination of the transaction in a selectable format.

Further, when acquiring the contract address of the smart contract, the address acquisition unit 223 may also transmit the public address of the challenger so that the user can acquire, as the challenger, a list of contract addresses of smart contracts registered in the administrator terminal 40.

As for a content, such as images and the like, which supports the determination data, the determination data acquisition unit 226 generates a hash value for uniquely identifying the content, and transmits, to the blockchain network 55, only the generated hash value as the transaction. The actual content is transmitted to the administrator terminal 40 together with the hash value. The images include, for example, meal images taken by the challenger, an image of body measurement results taken by the challenger, and the like. Use of the hash value eliminates the need to record, directly in the blockchain network 55, such a content that the size is large and the cost (GAS cost) is quite high.

The token issuance information generation unit 227 generates token issuance information for issuing a token in the blockchain network 55.

The token issuance information includes the total number of issued tokens, the name of the token, the unit notation of the token, and the like. Based on user input from the input unit 211, the token issuance information generation unit 227 acquires these pieces of information to generate the token issuance information.

Based on the token issuance information, the transaction generation unit 225 transmits a transaction to the blockchain network 55. When the transaction is approved in the blockchain network 55, a token contract is generated in the blockchain network 55. The fact that the token contract as a smart contract for managing the token balance is generated means that the token is issued.

The token contract has a contract address in the blockchain network 55. The token contract manages the token balance in the blockchain network 55 by recording a public address to own the issued token and the token balance status for the public address.

In the first embodiment, a thanks token contract 502 is generated as the token contract as illustrated in Fig. 7. The thanks token contract 502 will be described later.

The challenge app 80 in the challenger terminal 20 may also be able to implement the challenge setting unit 126 and the contract information generation unit 127 in the fund provider terminal 10. The challenge app 80 in the fund provider terminal 10 may also be able to implement the determination data acquisition unit 226 and the token issuance information generation unit 227 in the challenger terminal 20. In other words, the fund provider terminal 10 and the challenger terminal 20 are distinguished by the position as the fund provider and the position as the challenger, but the distinction between both can be arbitrarily replaced. Further, the fund provider and the challenger may be the same person.

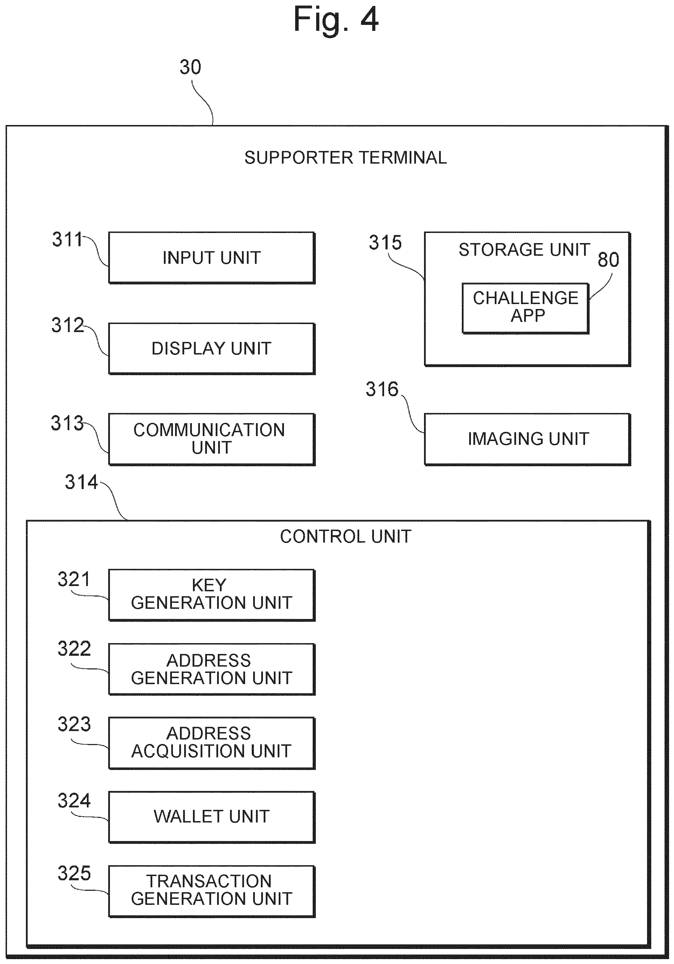

A block diagram of the supporter terminal 30 is illustrated in Fig. 4. The supporter terminal 30 is an information processing terminal having a function to perform communication with the administrator server and the blockchain network 55 through the network N. For example, the supporter terminal 30 may be, but not limited to, a smartphone, a PC, a tablet, or the like.

The supporter terminal 30 includes an input unit 311, a display unit 312, a communication unit 313, a control unit 314, a storage unit 315, and an imaging unit 316.

The input unit 311, the display unit 312, the communication unit 313, and the imaging unit 316 are the same as the input unit 111, the display unit 112, the communication unit 113, and the imaging unit 116. The supporter terminal 30 transmits own user identification information to the administrator terminal 40. The challenge app 80 is stored in the storage unit 315.

The control unit 314 includes a CPU and a memory. The control unit 314 functions as a key generation unit 321, an address generation unit 322, an address acquisition unit 323, a wallet unit 324, and a transaction generation unit 325 when the challenge app 80 (program) stored in the storage unit 315 is read into a RAM and executed by the CPU.

The key generation unit 321, the address generation unit 322, the address acquisition unit 323, the wallet unit 324, and the transaction generation unit 325 have the same functions as the key generation unit 121, the address generation unit 122, the address acquisition unit 123, the wallet unit 124, and the transaction generation unit 125.

The public address of a supporter generated by the address generation unit 322 is stored in the storage unit 315. The supporter terminal 30 functions as a terminal capable of sending/receiving a cryptocurrency in the blockchain network 55. The supporter terminal 30A is the same as the supporter terminal 30.

The challenge app 80 in the supporter terminal 30 may also be able to implement the challenge setting unit 126 and the contract information generation unit 127 in the fund provider terminal 10 or implement the determination data acquisition unit 226 and the token issuance information generation unit 227 in the challenger terminal 20. In other words, the distinction among the fund provider terminal 10, the challenger terminal 20, and the supporter terminal 30 can be arbitrarily replaced with one another depending on the challenge.

The administrator terminal 40 includes an input unit 411, a communication unit 413, a control unit 414, and a storage unit 415. For example, the administrator terminal 40 is a server device. The input unit 411 and the communication unit 413 are the same as the input unit 111 and the communication unit 113.

In the storage unit 415, a challenge DB 4151, a member DB 4152, and a content DB 4153 are stored as databases (DBs).

The control unit 414 includes a CPU and a memory. The control unit 414 functions as a key generation unit 421, an address generation unit 422, an address acquisition unit 423, a wallet unit 424, a transaction generation unit 425, a challenge setting unit 426, a contract information generation unit 427, a token issuance information generation unit 428, a site providing unit 431, a member registration unit 432, and a content receiving unit 433 when a program stored in the storage unit 415 is read into a RAM and executed by the CPU.

In the challenge DB 4151, information about challenges is stored. In the first embodiment, information about a challenge tried by each user in the challenge assistance system 1 is stored for each challenge in the challenge DB 4151. The information about the challenge includes a contract address of the challenge contract 501 that defines the challenge, contract terms, and the like.

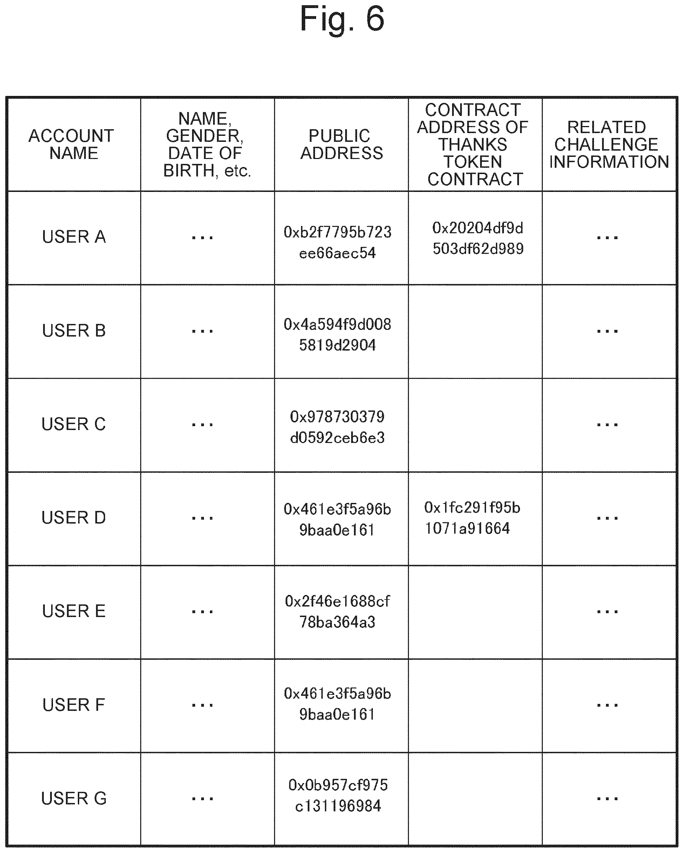

In the member DB 4152, information about members of the challenge assistance system 1 is stored. In the first embodiment, it is desired that user identification information such as name, gender, date of birth, and account name, the public address of each user, related challenge information related to each member, and the like should be registered in the member DB 4152 as illustrated in Fig. 6. Note that each public address illustrated in Fig. 6 is a fictional address for description, and the number of digits in the address is different from that in an actual address used in the Ethereum. The same also applies to the other drawings.

When the user has the thanks token contract 502 in the blockchain network 55, a contract address of the thanks token contract 502 may also be registered.

In the related challenge information, the contract address of a smart contract that defines a challenge related to the user and a participation attribute to a campaign are stored. In the participation attribute, for example, "fund provider" indicative of a creator of the challenge, "challenger" indicative of a challenger for the challenge, or the like is set. Note that information about any challenge related to the user can be stored in the related challenge information.

In the content DB 4153, information about the content such as images and sound as the evidence of activities to achieve a challenge in the challenge assistance system 1 is stored. In the first embodiment, it is desired that the content and a hash value generated from the content should be stored in the content DB 4153. The hash value is information for uniquely identifying the content. Use of the hash value eliminates the need to record an image file and the like in the blockchain network 55. Therefore, the content itself is kept secret without being made public by the blockchain network 55. Note that data different from the hash value may also be used as long as the content is uniquely identifiable from the data recorded in the blockchain network 55.

The key generation unit 421, the address generation unit 422, the address acquisition unit 423, the wallet unit 424, and the transaction generation unit 425 have the same functions as the key generation unit 121, the address generation unit 122, the address acquisition unit 123, the wallet unit 124, and the transaction generation unit 125.

A secret key generated by the key generation unit 421 is stored in the storage unit 415. The public address of an administrator generated by the address generation unit 422 is stored in the storage unit 415. The fund provider terminal 10 can acquire the public address of the administrator by the address acquisition unit 123 of the fund provider terminal 10.

The challenge setting unit 426 and the contract information generation unit 427 are the same as the challenge setting unit 126 and the contract information generation unit 127. The challenge setting unit 426 and the contract information generation unit 427 enables the administrator to create a challenge. Note that upon creation of a challenge, the administrator terminal 40 may receive contract information generated externally, and transmit a contract generation transaction to the blockchain network 55 to create the challenge.

Like the token issuance information generation unit 227, the token issuance information generation unit 428 enables the administrator to issue a token in the blockchain network 55. Note that upon issuance of a token, the administrator terminal 40 may receive token issuance information generated externally, and transmit the token issuance information to the blockchain network 55 to issue the token.

The site providing unit 431 provides, on the network N, a website for using the challenge assistance system 1. On the site, the fund provider, the challenger, and a refunder as a destination to which the funds are refunded can register as members. Therefore, the site providing unit 431 transmits a web page written in HTML (HyperText Markup Language) or the like to the terminal used by each user, and receives user input results and the like from the terminal.

The member registration unit 432 receives user identification information from the terminal of each user, and registers the user identification information in the member DB 4152. In the first embodiment, when member information is transmitted from the terminal according to user operations on a member registration screen provided by the site providing unit 431, the member registration unit 432 registers data in the member DB 4152 based on the user identification information received.

When a public address is received together with user identification information from a terminal with the challenge app 80 downloaded thereto, the member registration unit 432 stores the public address in the member DB 4152 identified by the user identification information.

The content receiving unit 433 receives a content such as image data and sound data from the challenger terminal 20 or the determination data transmission terminal 60 to be described later, and stores the content in the content DB 4153. The content receiving unit 433 receives a hash value for uniquely identifying the content together with the content, and stores the received hash value and content in the content DB 4153.

In the blockchain network 55, the challenge contract 501, the thanks token contract 502, and a cheering token contract 503 are provided.

The challenge contract 501 is a smart contract generated based on contract information generated by the contract information generation unit 127 of the fund provider terminal 10 or the contract information generation unit 427 of the administrator terminal 40.

The thanks token contract 502 is a smart contract generated based on token issuance information generated by the token issuance information generation unit 227 of the challenger terminal 20.

The cheering token contract 503 is a smart contract generated based on token issuance information generated by the token issuance information generation unit 428 of the administrator terminal 40.

A dapp (Decentralized application) having a function to increase the possibility of letting a user achieve a challenge is built by the challenge contract 501, the thanks token contract 502, and the cheering token contract 503 provided on the blockchain network 55. Each user uses the dapp by paying a prescribed usage fee via a client terminal such as the fund provider terminal 10 or the challenger terminal 20. The usage fee in the Ethereum is GAS.

The determination data transmission terminal 60 includes an input unit 611, a display unit 612, a communication unit 613, a control unit 614, a storage unit 615, and an imaging unit 616.

The input unit 611, the display unit 612, the communication unit 613, and the imaging unit 616 are the same as the input unit 111, the display unit 112, the communication unit 113, and the imaging unit 116. The determination data transmission terminal 60 transmits stored user identification information to the administrator terminal 40. A challenge app 81 is stored in the storage unit 615.

The control unit 614 includes a CPU and a memory. The control unit 614 functions as a key generation unit 621, an address generation unit 622, an address acquisition unit 623, a wallet unit 624, a transaction generation unit 625, a determination data acquisition unit 626, and a key concealment unit 627 when the challenge app 81 (program) stored in the storage unit 615 is read into a RAM and executed by the CPU.

The key generation unit 621, the address generation unit 622, the address acquisition unit 623, and the transaction generation unit 625 have the same functions as the key generation unit 121, the address generation unit 122, the address acquisition unit 123, and the transaction generation unit 125. The determination data acquisition unit 626 has the same function as the determination data acquisition unit 226.

The key concealment unit 627 has a function to disable a user of the determination data transmission terminal 60 to transfer a secret key generated by the key generation unit 621 to any other terminal. A general wallet for a cryptocurrency has a function to provide, to a user, a display of information necessary for transfer and backup of a secret key. For example, the wallet displays the secret key as an image or a mnemonic. The key concealment unit 627 restricts the function to provide, to the user, a display of information necessary for transfer of the secret key and backup of the secret key to restrict the transfer of the secret key.

The reason for restricting the transfer of the secret key will be described. In the blockchain network 55, whether or not a transaction was really sent by a sender is identified by verifying each node of the blockchain network 55 as to whether or not the transaction is signed with a secret key of the sender. Signing the transaction with the secret key of the sender means that the transaction was transmitted by a transaction owner.

If the secret key is transferred, determination data can be sent to the challenge contract 501 using any other terminal or software. If the determination data signed with an own secret key is sent by using any other terminal or software, it will be indistinguishable whether the determination data in the challenge contract 501 is data from the terminal owned by the user or data from any other terminal or software.

The determination data transmission terminal 60 can restrict the transfer of the secret key by the key concealment unit 627 to avoid such a situation that the determination data is transmitted from any terminal other than the determination data transmission terminal 60. Therefore, the smart contract can confirm that the determination data signed with the secret key of the determination data transmission terminal 60 is sent from the determination data transmission terminal 60.

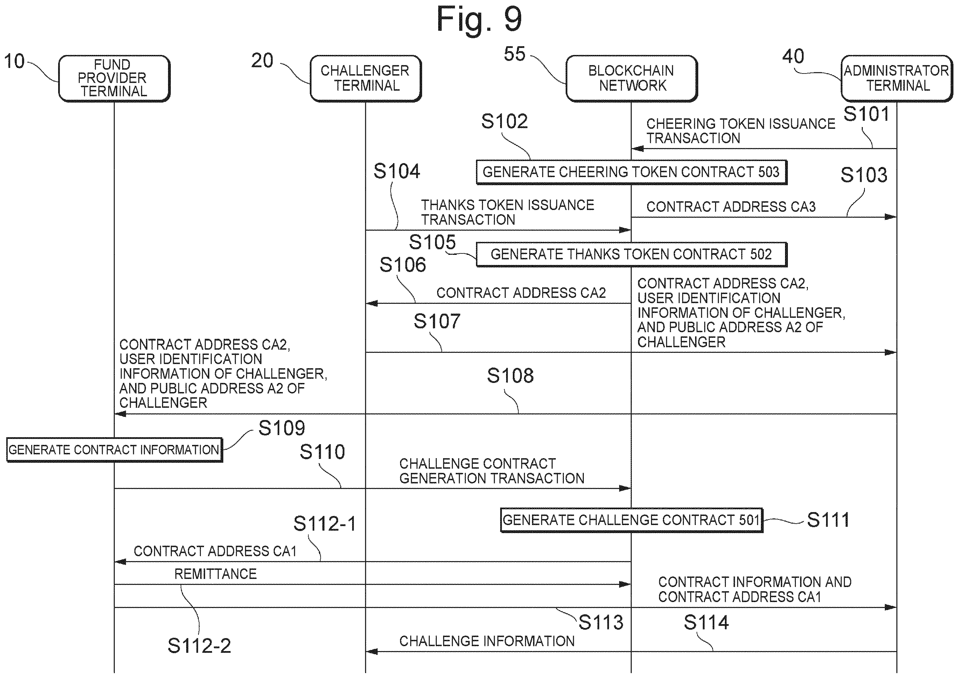

Referring to sequence diagrams of Fig. 9 and Fig. 10, and Fig. 11 to Fig. 21, the operation of the challenge assistance system 1 will be described.

In step S101, the administrator terminal 40 transmits, to the blockchain network 55, a transaction to generate the cheering token contract 503.

In step S102, the transaction is approved by the blockchain network 55, and the cheering token contract 503 is generated in the blockchain network 55.

In step S103, the blockchain network 55 transmits a contract address CA3 of the cheering token contract 503 to the administrator terminal 40.

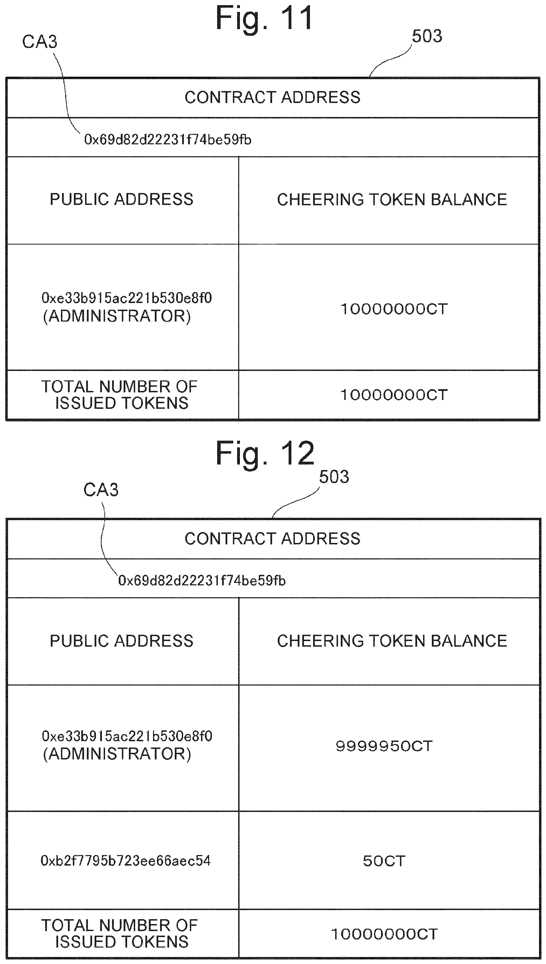

An initial example of the cheering token contract 503 generated in the blockchain network 55 is illustrated in Fig. 11. The currency unit of cheering tokens is set as "CT." A public address of "Ox69d..." is the contract address CA3 of the cheering token contract 503. A public address of "Oxe33..." is a public address of the administrator corresponding to a secret key stored in the administrator terminal 40.

The cheering token contract 503 allocates, to another user, a cheering token to exchange with ETHER. When the cheering token is allocated, the cheering token contract 503 adds a public address of a holder of the cheering token, and manages the cheering token balance about the public address as illustrated in Fig. 12.

In step S104, the challenger terminal 20 transmits, to the blockchain network 55, a transaction to generate the thanks token contract 502.

In step S105, the transaction is approved by the blockchain network 55, and the thanks token contract 502 is generated in the blockchain network 55.

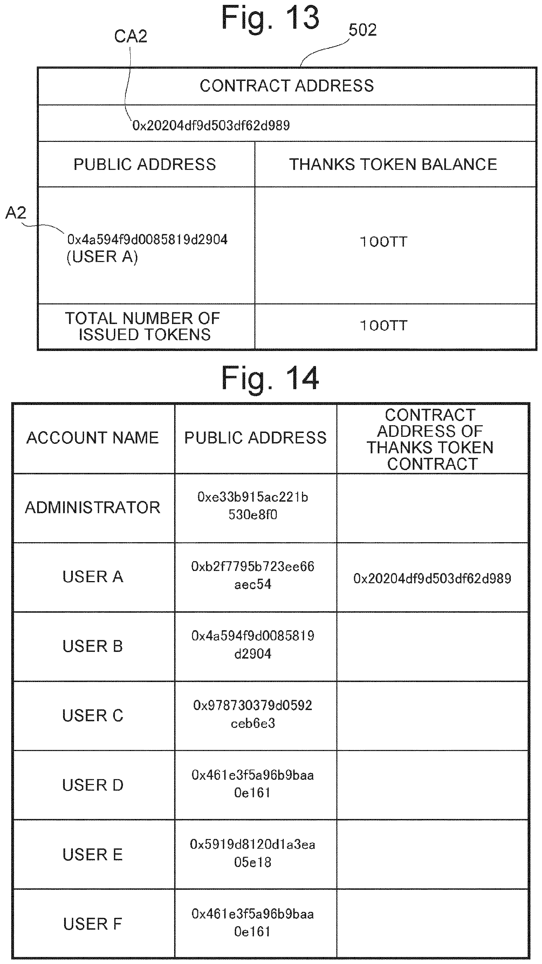

An initial example of the thanks token contract 502 generated in the blockchain network 55 is illustrated in Fig. 13. The currency unit of cheering tokens is set as "TT." A public address of "Ox202..." is a contract address CA2 of the thanks token contract 502. A public address of "Oxb2f..." is a public address A2 of the challenger corresponding to the secret key stored in the challenger terminal 20. Here, a user having the public address of "Oxb2f..." is set as user A.

In step S106, the blockchain network 55 transmits the contract address CA2 of the thanks token contract 502 to the challenger terminal 20.

In step S107, the challenger terminal 20 transmits, to the administrator terminal 40, the contract address CA2, user identification information of the challenger, and the public address A2 of the challenger.

In step S108, the administrator terminal 40 transmits, to the fund provider terminal 10, the contract address CA2, the user identification information of the challenger, and the public address A2. By this process, the fund provider terminal 10 can store the contract address CA2 of the thanks token contract 502 and the public address A2 of the challenger in association with each other. As illustrated in Fig. 14, each account name and each public address are stored in association with each other in the fund provider terminal 10. In Fig. 14, when there is no thanks token contract corresponding to a public address, the public address of the thanks token contract is left blank.

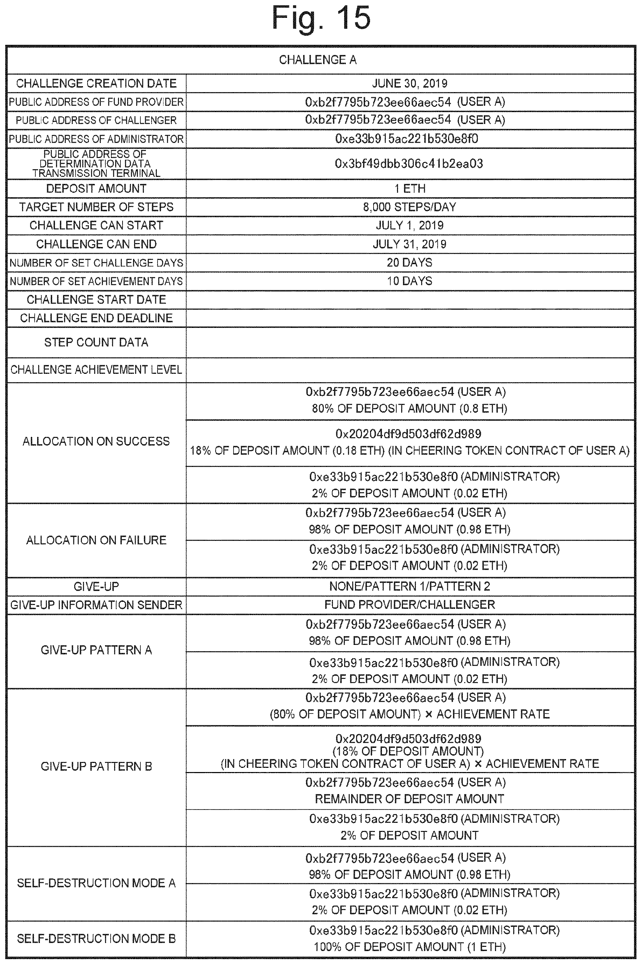

In step S109, the fund provider terminal 10 generates contract information based on the contract address CA2, the user identification information of the challenger, the public address A2, the contract terms, and the like. An example of the contract information generated at this time is illustrated in Fig. 15.

The contract information illustrated in Fig. 15 is contract information when user A creates challenge A as a challenge to walk a certain number of steps or more a day. The content of the contract information is as follows.

The challenger can do the challenge in a period from July 1, 2019 to July 31, 2019. The target number of steps is 8,000 steps per day. The challenger selects 20 days from the period. The challenge is achieved when the challenger walks 8,000 steps a day for consecutive or intermittent 10 days or more during the days. The deposit amount is 1 ETH.

Step count data is used to determine the success or failure of the challenge. The step count data is stored in the smart contract.

An item of "public address of determination data transmission terminal" is provided in the contract information. The public address corresponding to this item corresponds to the public address of the determination data transmission terminal 60. The public address of the determination data transmission terminal 60 is included in the user identification information of the challenger, included in the user identification information of the fund provider, or the like. When the determination data transmission terminal 60 is not used, no special setting is made.

Items of "challenge start date" and "challenge end deadline" are provided in the challenge information. The challenge start date is the date on which the challenger started the challenge. For example, the challenge start date can be set to the date on which the determination data was first transmitted.

The challenge end deadline is a deadline to end the challenge. In Fig. 15, the challenge must be achieved in 20 days from the challenge start date. If the challenge start date is July 5, 2019, the challenge end deadline will be July 24, 2019.

An item of "challenge achievement level" is provided in the contract information. This item is provided for the challenge contract 501 to calculate and store the challenge achievement level from the step count data and the target number of steps in the process of the challenge. For example, the achievement level can be calculated as a ratio of the current number of steps to the target number of steps to be achieved in the challenge.

A deposit is allocated to an allocation destination based on allocation settings determined respectively when the challenge is successful and when the challenge is unsuccessful. The allocation destination and the allocation amount can be set freely. The allocation amount may be set as a percentage of the deposit amount. The allocation amount may also be set directly as the amount of ETHER to be allocated. The administrator fee is deducted, for example, as 2 percent of the deposit.

When an allocation destination is specified, the public address of the allocation destination can be directly specified. For example, the public address can be acquired from an image read by the imaging unit 116 of the fund provider terminal 10 and set as the allocation destination. A public address stored in the storage unit 115 of the fund provider terminal 10 as illustrated in Fig. 14 can also be used.

Fund allocation states include refund, donation, and forfeiture, respectively, and allocation settings specified to match the total amount specified in refund, donation, and forfeiture to the fund amount are sent from the fund provider terminal 10. In the case of refund, any refund destination holding an Ethereum public address and any amount can be specified. In the case of donation, any donation destination holding an Ethereum public address and any amount can be specified. The administrator fee may be paid to the administrator at the time of success/failure/termination of the challenge.

The public address stored in the storage unit 115 can be synchronized with the user identification information stored in the administrator terminal 40, and when the address of any other user is changed, a new address can be automatically acquired.

Upon creation of a challenge, it can be determined whether or not the challenge can be interpreted based on information indicative of the termination of the challenge.

As the termination of the challenge, there are termination due to give-up and termination due to self-destruction. The termination due to give-up is such a termination that the fund provider or the challenger gives up on the achievement of the challenge and transmits information indicative of the termination of the challenge to the smart contract as a transaction.

As the termination due to self-destruction, there is a case when a trouble occurs, such as no funds from the challenge contract 501 for some reason, and the administrator causes the self-destruction of the challenge contract 501 in response to a request from the user, when ordered, required, or requested by the police, the law, the court, or the government agency, or when the administrator decided to be necessary independently. In the case of the termination due to self-destruction, the administrator transmits a transaction to the challenge contract 501 to cause the self-destruction of the challenge contract 501.

As the allocation of the deposit in the case of give-up, there are give-up pattern A in which the remainder after deduction of the administrator fee is allocated to a predetermined allocation destination, give-up pattern B in which the deposit is allocated to the same allocation destination as that at the time of success of the challenge in consideration of a challenge achievement level before give-up and the remainder after deduction of the administrator fee is returned to the fund provider, and the like.

When causing the self-destruction of the challenge contract 501, the deposit is allocated in self-destruction mode A to partially return the funds to the fund provider, in self-destruction mode B to send the full amount to the administrator, and the like.

In step S110, the fund provider terminal 10 transmits, to the blockchain network 55, a contract generation transaction to generate the challenge contract 501 based on the contract information.

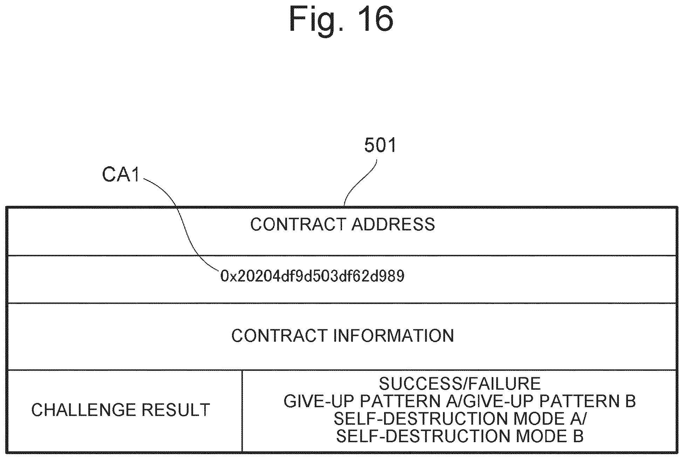

In step S111, the contract generation transaction is approved by the blockchain network 55, and the challenge contract 501 is generated in the blockchain network 55. In addition to the contract information described with reference to Fig. 15, the generated challenge contract 501 includes a contract address CA1 of the challenge contract 501 indicated as "0x202..." in Fig. 16 and challenge result data. The challenge result is information indicating that a deposit is allocated according to any of the following results: Success, failure, give-up pattern A, give-up pattern B, self-destruction mode A, or self-destruction mode B.

In step S112-1, the blockchain network 55 transmits the contract address CA1 of the challenge contract 501 to the fund provider terminal 10. In step S112-2, the fund provider terminal 10 sends the deposit amount to the challenge contract 501 using the contract address CA1 as a destination. The challenge contract 501 holds the deposit amount as a balance associated with the contract address CA1.

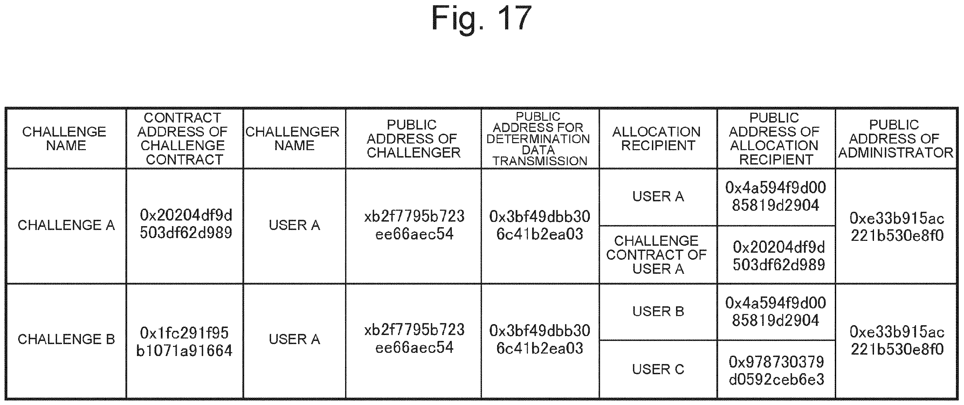

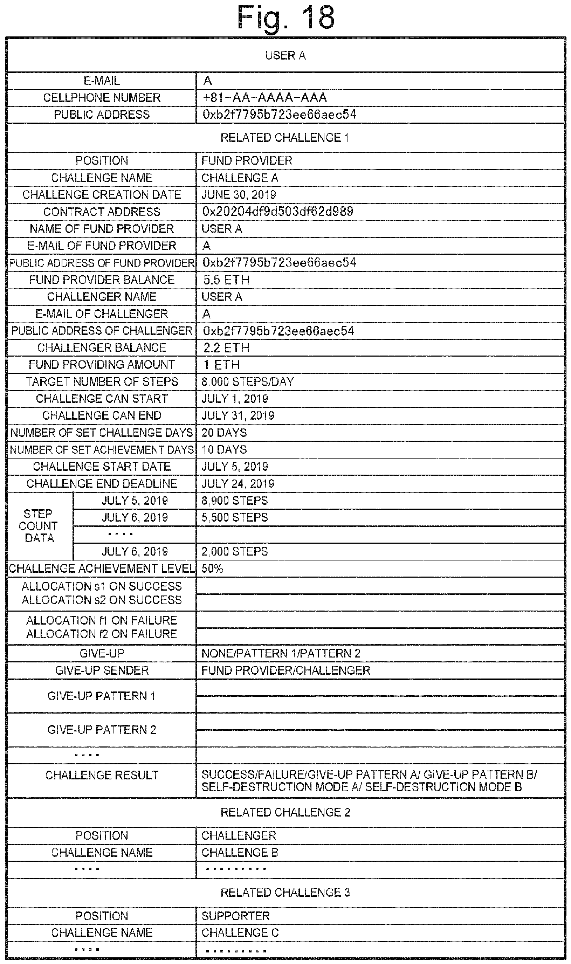

In step S113, the fund provider terminal 10 transmits the contract information and the contract address CA1 to the administrator terminal 40. As illustrated in Fig. 17, the administrator terminal 40 records information on each challenge in the challenge DB 4151. Note that information stored in an item of "allocation recipient" may also include a smart contract such as the challenge contract 501. Further, information stored in an item of "public address of allocation recipient" may include the contract address of the smart contract. Further, as illustrated in Fig. 18, the administrator terminal 40 records, in the member DB 4152, user identification information of each user and a challenge associated with the user.

In step S114, the administrator terminal 40 transmits, to the challenger terminal 20, challenge information including the contract information and the contract address CA1. Thus, the challenger can get information on the content of the challenge, and to which contract address the determination data should be transmitted.

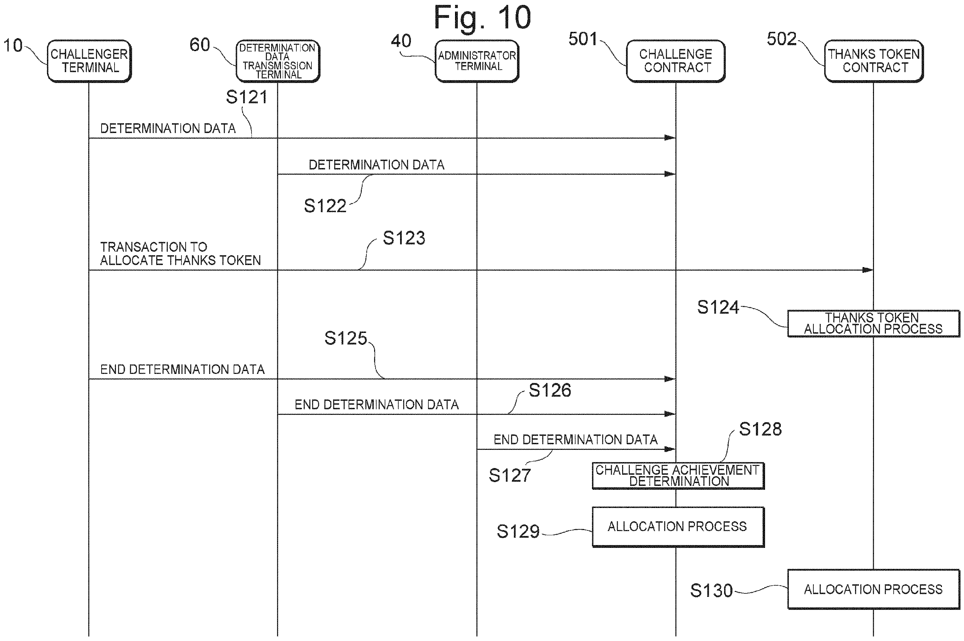

In step S121 illustrated in Fig. 10, the challenger transmits the determination data from the challenger terminal 20 to the challenge contract during the challenge period. The challenger terminal 20 may not be necessarily used to transmit the determination data. Like in step S122, the determination data may be transmitted to the challenge contract by using the determination data transmission terminal 60.

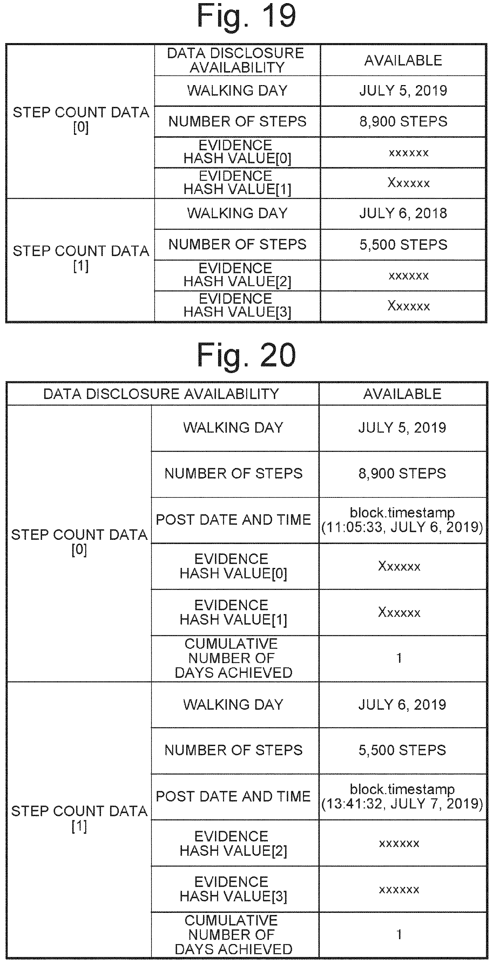

An example of the determination data transmitted from the challenger terminal 20 to the challenge contract is illustrated in Fig. 19. In Fig. 20, a data structure of the determination data transmitted from the challenger terminal 20 to the challenge contract and stored in the challenge contract 501 by a transmitted transaction is illustrated. As the data posting date and time, the block generation date and time of the blockchain network 55 in which the transaction is stored is added to the data in Fig. 19. Further, the number of days achieved is recorded by the challenge contract 501.

In step S123, the challenger transmits, to the thanks token contract, a transaction to allocate a thanks token to each supporter who supports the challenge of the challenger.

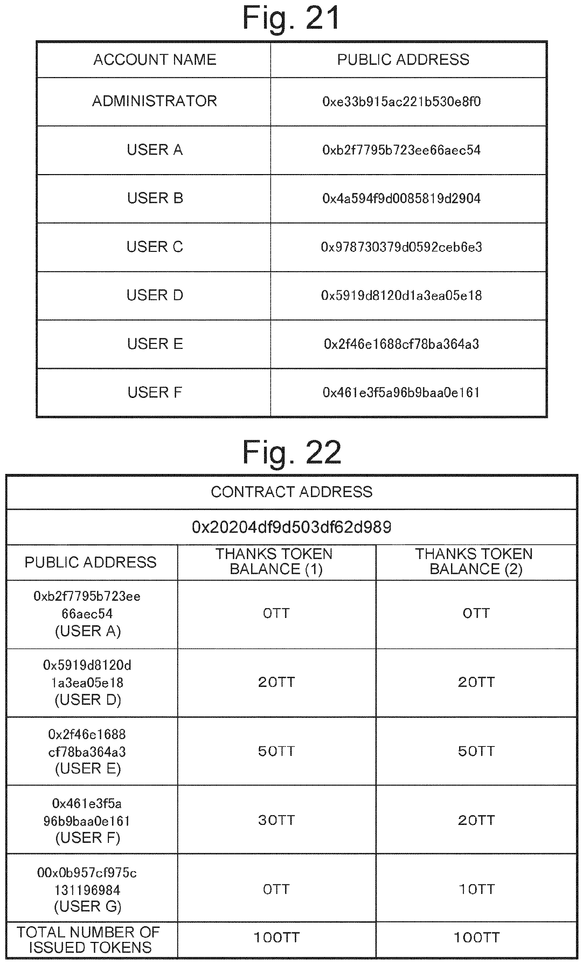

As illustrated in Fig. 21, the challenger acquires, from the administrator terminal 40, addresses of users of the challenge assistance system 1, each of which installs the challenge app 81 on the supporter terminal 30, and stores the addresses. The challenger can allocate the thanks token to each address.

In step S124, the balance of the thanks token contract 502 is updated when the transaction is approved by the blockchain network 55. The thanks token is allocated to each supporter by updating the balance of the thanks token contract 502.

The supporter to which the thanks token was allocated from the challenger can reallocate the thanks token to any other supporter. When the thanks token is reallocated, only the supporter as the source of reallocation only has to know the public address of a reallocation destination. In other words, the thanks token can circulate from one supporter to another.

An example of the balance of the thanks token contract 502 after the allocation and reallocation of thanks tokens is illustrated. The balance of the user A as the challenger is 0. As illustrated in Fig. 22, the challenger terminal 20 acquired respective public addresses of user D, user E, and user F, and allocated thanks tokens to the respective users. The user A allocated 20TT to the user D, 50TT to the user E, and 30TT to the user F. As a result, the thanks token balance is indicated as thanks token balance (1) in Fig. 22. After that, the result of reallocation of 10TT from the user F to user G having the supporter terminal 30A initially not known by the user A is thanks token balance (2) in Fig. 22.

In step S125, the challenger terminal 20 transmits end determination data to the challenge contract 501. Like in step S126, the end determination data may also be transmitted from the determination data transmission terminal 60. Alternatively, like in step S127, the end determination data may be transmitted from the administrator terminal 40.

The end determination data is used by the challenge contract 501 to determine success/failure of the challenge. For example, such a challenge as to walk 8,000 steps or more a day for 10 days during 20 days from July 5 to July 24 according to the contract information of Fig. 15 is assumed. It is also assumed that the challenger walked 8,000 steps or more for 9 days from July 5 to July 13. In this case, when 10,000 steps is sent as step count data on July 14, the success of the challenge is the end determination.

Data of 10,000 steps on July 14 becomes "step count data for end determination." At the time when a transaction to transmit the step count data for end determination is approved by the blockchain network 55, the success of the challenge is confirmed.

Further, it is assumed that the challenger walked 8,000 steps or more for 9 days from July 5 to July 13 in the challenge with the same conditions. After that, it is assumed that the challenger walked less than 8,000 steps from July 14 to July 23. In this case, when the challenger sends 5,000 steps as the number of steps on July 24, 5,000 steps becomes "step count data for end determination," and the failure of the challenge becomes the end determination.

When the challenger hesitates or forgets to send data of 5,000 steps on July 24, the challenge contract 501 cannot determine the success/failure of the challenge.

The smart contract may also take in date information at a block time recorded by a miner mining the block in the blockchain network 55. Further, the date information may be taken in by using any other function of the blockchain network. The smart contract in the first embodiment needs some date information from the outside. This is because when step count data is not sent even after July 24, the setting of considering that the challenge is a failure cannot be made in the challenge contract 501. A person concerned such as the fund provider or the administrator may also send date information to the smart contract.

In the example described above, when the data of 5,000 steps on July 24 is not sent, the administrator may use email, a notice on the app, or the like on July 25 or 26 to let the fund provider or the challenger know that step count data is not sent. The administrator can know data status in the challenge contract 501 by information from the app or by directly referring to the challenge contract 501. The challenger is originally required to transmit all steps during the challenge period at the right timings. However, when the challenger does not fulfill the requirements, the unsent steps are handled in the challenge contract 501 to be zero when a certain grace period has passed after the end of the challenge period to be described later. When the fund provider that received the notice email described above sends date data indicating that "today is July 26" to the challenge contract 501 as a transaction, since the challenge contract 501 handles the unsent steps on July 24 from the challenger to be zero, the challenge contract 501 determines the failure of the challenge. In this case, the information about the date from the fund provider becomes "date data for end determination."

When someone transmits "end determination data" as mentioned above and the "end determination data" is taken in the blockchain network 55, the challenge contract 501 automatically starts operating as determined in advance.

A challenge achievement determination can be made by sending the end determination data to the challenge contract 501. In step S128, the challenge contract 501 of the blockchain network 55 performs a challenge achievement determination process.

In step S129, the challenge contract 501 performs an ETHER allocation process based on the determination result.

The ETHER allocation process by the challenge contract 501 is performed by the challenge contract 501 generating an internal transaction in the Ethereum. The internal transaction in the Ethereum is a transaction generated by the smart contract using a transaction received from the challenger or the like as a trigger.

In step S130, an ETHER allocation process to allocate ETHER to each supporter based on the thanks token balance is performed by the thanks token contract 502 with ETHER allocated from the challenge contract 501.

The allocation of ETHER based on the thanks token balance will be described by taking, as an example, a case based on the thanks token balance (2) in Fig. 22. It is assumed that the challenge contract 501 holds a deposit of 1 ETH. It is then assumed that 0.8 ETH is allocated to the thanks token contract 502 from the deposit of 1 ETH. In this case, 0.8 ETH is allocated according to the token equity of each of user D, user E, user F, and user G, respectively, that is, 0.16 ETH to user D, 0.4 ETH to user E, 0.16 ETH to user F, and 0.08 ETH to user G.

In addition to ETHER, the thanks token contract 502 may also allocate various tokens in the same way when the tokens are sent to the thanks token contract 502.

For price fluctuation risk hedging of a cryptocurrency-based deposit amount deposited by the fund provider to the challenge contract 501, a cryptocurrency which can always be exchanged for a legal currency at the same rate and whose value does not fluctuate may be used upon creation of the challenge contract 501 to build this challenge assistance system 1.

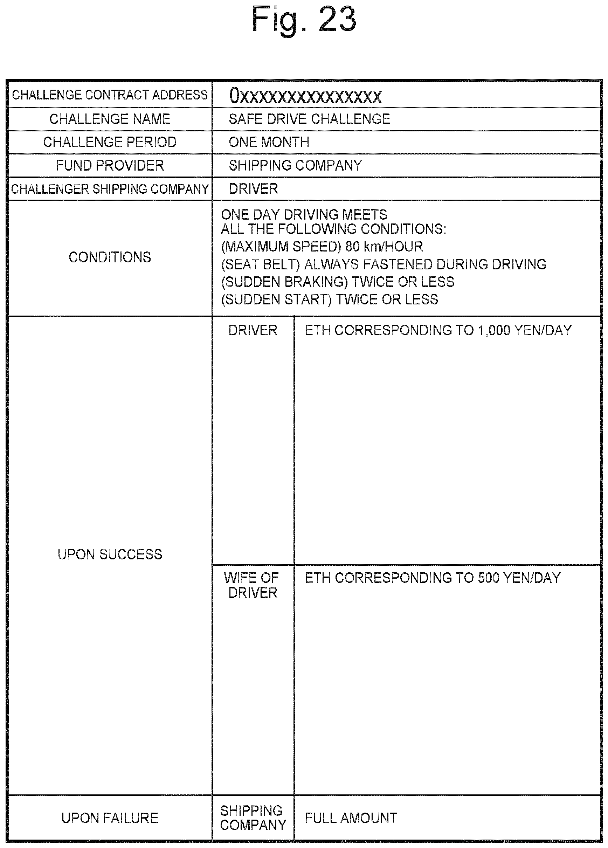

The challenge defined by the challenge contract 501 may also be any other type of challenge different from the challenge related to the number of steps. Referring to Fig. 23 to Fig. 26, challenge variations will be described.

In Fig. 23, the content of a smart contract for a safe drive challenge is illustrated. Determination data is information from a car or a smartphone owned by a driver, such as car speed, whether or not a seat belt is fastened, sudden braking, and sudden start. For acquisition and transmission of the determination data, an loT device having a wallet function may be used. The information may be transferred from the loT device to the smartphone or the like to send, to the blockchain network, a transaction signed with a secret key of a wallet app built in the smartphone. A shipping company may create a smart contract for a challenge to send ETHER or a token automatically to each truck driver when one day driving meets determination conditions.

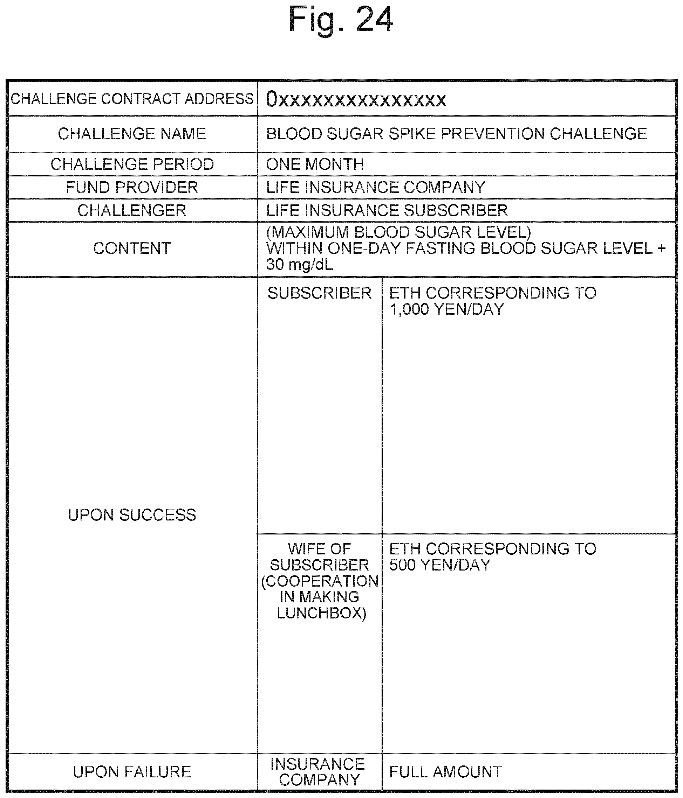

In Fig. 24, the content of a smart contract for a challenge to prevent a blood sugar spike is illustrated. The blood sugar spike that causes blood sugar to rise sharply becomes a problem. For example, when a large amount of sugar is ingested at lunch after skipping breakfast, a blood sugar spike occurs. The blood sugar spike can be prevented by devising ways such as to eat vegetables and seaweed first and not to keep hungry for a long time. Blood-sugar level data from equipment attached to an arm to be able to continuously measure the blood-sugar level may be transferred to a smartphone or the like to send, to the blockchain network, a transaction signed with a secret key of a wallet app built in the smartphone. An insurance company may create a smart contract as in Fig. 24 for an insurance subscriber to learn such a lifestyle as not to raise the blood-sugar level sharply. The insurance subscriber does not take it seriously unless there is an economic reward in the short term. First, a lecture on a method of not causing blood sugar spikes may be given before start of the challenge.

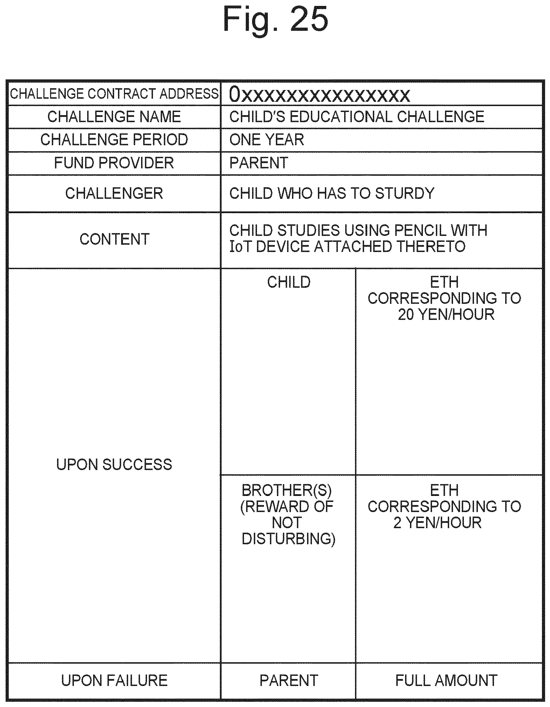

In Fig. 25, the content of a smart contract for a child's educational challenge is illustrated. When a child studies using a pencil with an loT device attached thereto, information indicating that the child studied for a certain period of time is transmitted from the loT device to the smart contract to send, to a wallet of the loT device or a wallet for the exclusive use of the child, ETHER or a token usable in an online game as a reward. When ETHER is sent to the loT device, the color of the loT device changes according to the accumulated ETHER. The child is aware that an allowance increases when studying a lot by looking at color changes, increasing motivation for studying. The reward amount should be adjusted according to the financial level of the parents. Upon success, some of ETHER may also be sent to brother(s) not to disturb the child's studies.

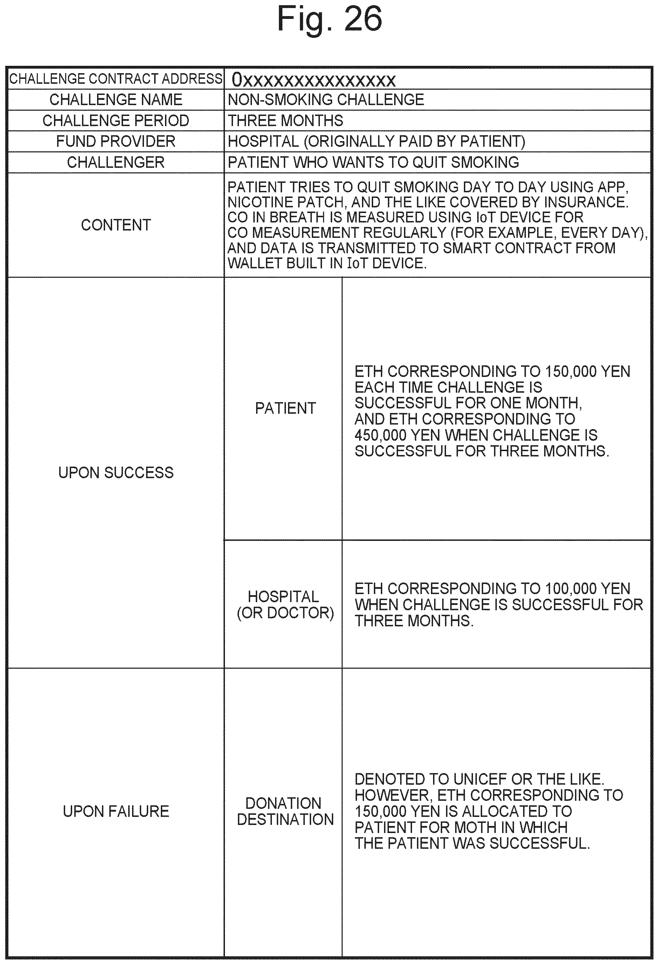

In Fig. 26, the content of a smart contract for a non-smoking challenge is illustrated. Self-paying treatment rather than treatment by insurance coverage is assumed. A doctor prescribes, in hospital, a non-smoking challenge of 600,000 yen for a patient who wants to quit smoking. The patient pays 600,000 yen to the hospital. In the hospital, a smart contract for the non-smoking challenge is created using ETHER which is worth 550,000 yen as an amount after deducting a profit and expenses of 50,000 yen. The doctor may use equipment for measuring CO in the breath to judge whether or not the patient achieves to quit smoking. CO in the breath may be measured using an loT device for CO measurement to send the data from a wallet built in the loT device to the smart contract so as to cause the smart contract to determine succeed/failure of the non-smoking challenge. A higher-price non-smoking challenge may also be prescribed.

When the challenge assistance system 1 is up and running, the cryptocurrency is required for generation of various smart contracts such as the challenge contract 501, transmission of various transactions, and deposit of deposit amount in the blockchain network 55. The fund provider and the like purchase cryptocurrency from a cryptocurrency exchange or the like. Another example of a cryptocurrency purchase method will be described.

The fund provider, the challenger, the supporter, and the like may also purchase cryptocurrency or tokens at convenience stores and the like. Each purchaser purchases a gift card having information on cryptocurrency or a token. When part of the gift card is scratched off, two-dimensional barcode appears. The two-dimensional barcode is a secret key of a gift card wallet. A gift card issuer sends, to a public address of the gift card wallet in advance, cryptocurrency or a token for the same amount as that written on the gift card. It is assumed that the gift card issuer stores and manages only the public address of the gift card without storing secret key information of the gift card at all.

The purchaser reads the two-dimensional barcode using an own app to take the gift card wallet into the app. Since the user acquires the secret key information of the gift card wallet, the user can use the gift card wallet freely.

Otherwise, when the purchaser selects a gift card on an ATM screen in a convenience store and makes a deposit, two-dimensional barcode having the same secret key as above is issued. The gift card wallet may be taken into the app by reading the two-dimensional barcode using the app there.

In this case, the gift card issuer may create a secret key and make a remittance to a public address corresponding thereto only when requested from the ATM. In the case of a gift card made of paper or the like, the remittance has to be made to the gift card wallet in advance. Since secret key information is contained in the two-dimensional barcode and the gift card is virtually as variable as a cash voucher, the gift card must be handled with care.

At the time when the secret key of the gift card wallet is read, two wallets, i.e., a wallet originally owned and the gift card wallet, exist in the app of the purchaser. In other words, two secret keys used to manage the cryptocurrency are owned. The purchaser transmits all the balance of the gift card wallet to the originally owned wallet, and deletes the gift card wallet.

Otherwise, the purchaser tells a convenience store employee at a cash register that the purchaser wants cryptocurrency or a token. Then, when paying cash, the employee reads two-dimensional barcode of the wallet originally existing in the app of the purchaser. This is a public address of the purchaser. Own cryptocurrency or token of the convenience store may also be sent directly to the public address.

Otherwise, when the cryptocurrency and the public address of the purchaser are sent as a transaction from a convenience store terminal to the smart contract created by the gift card issuer, the cryptocurrency or token may be transmitted from the smart contract directly to the wallet of the purchaser.

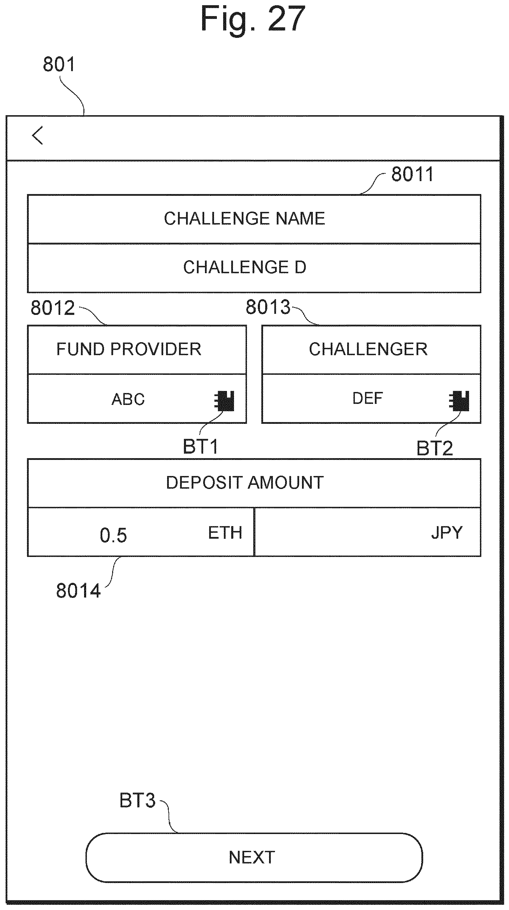

The transition of screens to be displayed on a terminal with the challenge app 80 installed thereon to generate a challenge contract by the challenge app 80 will be described with reference to Fig. 27 to Fig. 33.

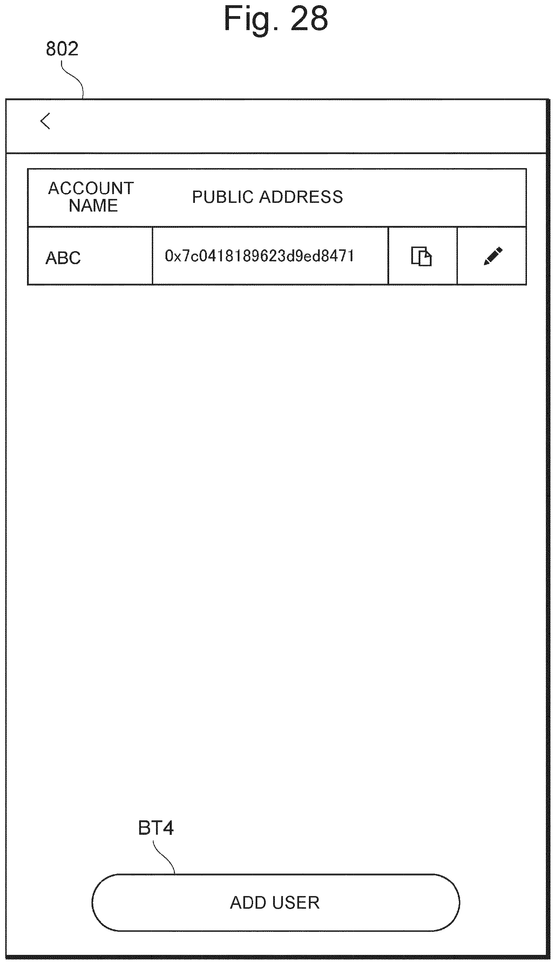

On a screen 801 of Fig. 27, a challenge field 8011, a fund provider field 8012, a challenger field 8013, and a deposit amount field 8014 are provided. In the challenge field 8011, a challenge name is input. In the fund provider field 8012 and the challenger field 8013, an account name of the fund provider is displayed. When a button BT1 or a button BT2 respectively in the fund provider field 8012 and the challenger field 8013 is selected, a screen 802 illustrated in Fig. 28 is displayed.

On the screen 802, the account name and a public address stored in the terminal are displayed in association with each other. The user can select each line to select the fund provider and the challenger. The user can select a button BT4 in Fig. 28 to add a pair of the account name and the address.

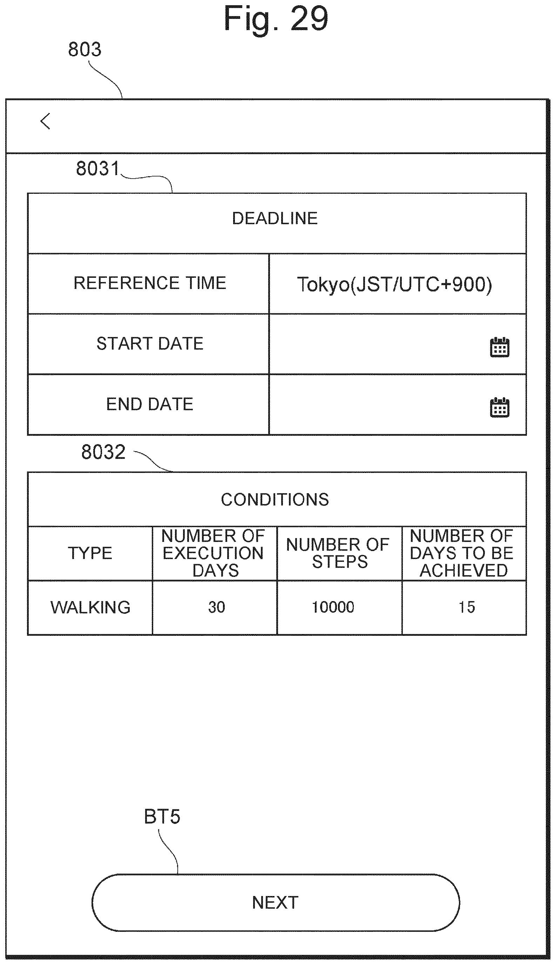

In the deposit amount field 8014 of Fig. 27, a deposit amount deposited by the fund provider is input. A screen 803 of Fig. 29 is displayed by the user selecting a button BT3 on the screen 801.

On the screen 803, a deadline field 8031 and a condition field 8032 are provided. In the deadline field 8031, a startable period and an endable period of the challenge are input. In the condition field 8032, the type of challenge, the challenge execution days, the number of steps, and the number of days to be achieved are input.

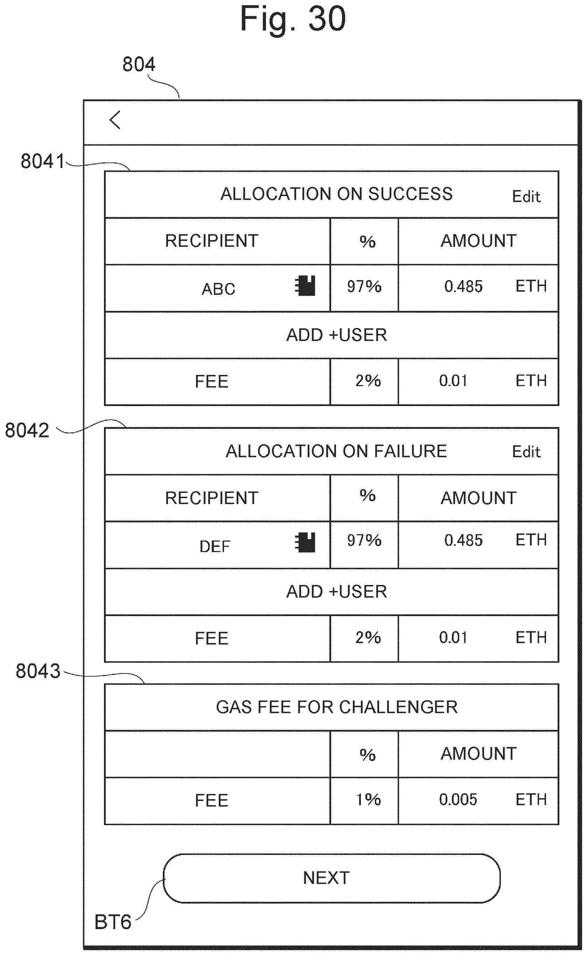

A screen 804 of Fig. 30 is displayed by the user selecting a button BT5 on the screen 803.

On the screen 804, an allocation-on-success field 8041, an allocation-on-failure field 8042, and a GAS fee field 8043 are provided.

In the allocation-on-success field 8041, the account name of a user who receives funds on success and an amount to receive are input. Further, 2% of the deposit amount is allocated as an administrator fee. The administrator fee may be set freely. The same applies to the allocation-on-failure field 8042. In the GAS fee-for-challenger field, an amount to subsidize a GAS fee required for the challenger to transmit step count information to the smart contract is input.

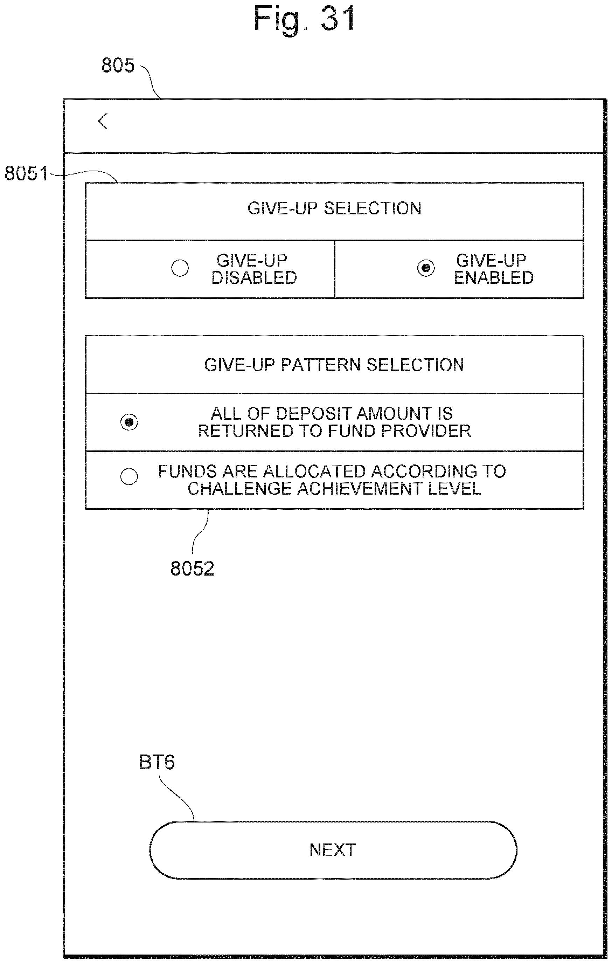

When the user selects a button BT6 on the screen 804, a screen 805 of Fig. 31 is displayed. The screen 805 has a give-up selection field 8051 and a give-up pattern selection field 8052. The user can select whether to enable give-up or not by selecting an item of the give-up selection field 8051. The user can select a deposit allocation method upon give-up by selecting an item of the give-up pattern selection field 8052.

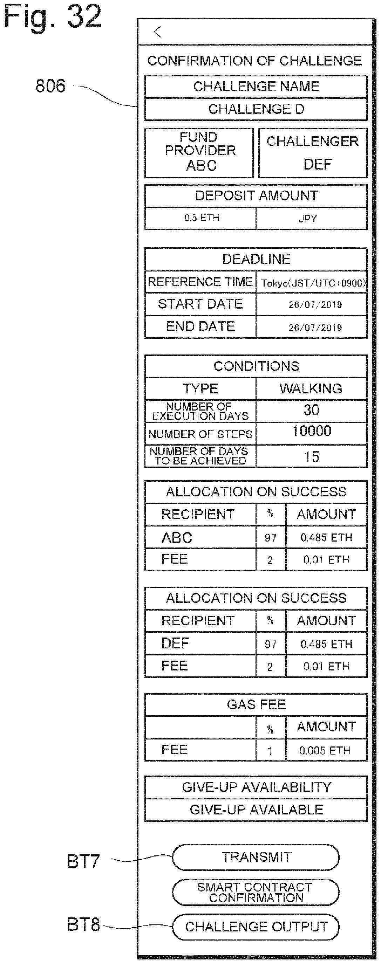

When the user selects a button BT6 on the screen 805, a screen 806 of Fig. 32 is displayed. On the screen 806, conditions set up so far are displayed as a list.

When the user selects a button BT7 on the screen 805, a transaction to generate, in the blockchain network 55, a challenge contract for managing the challenge is transmitted to the blockchain network 55.

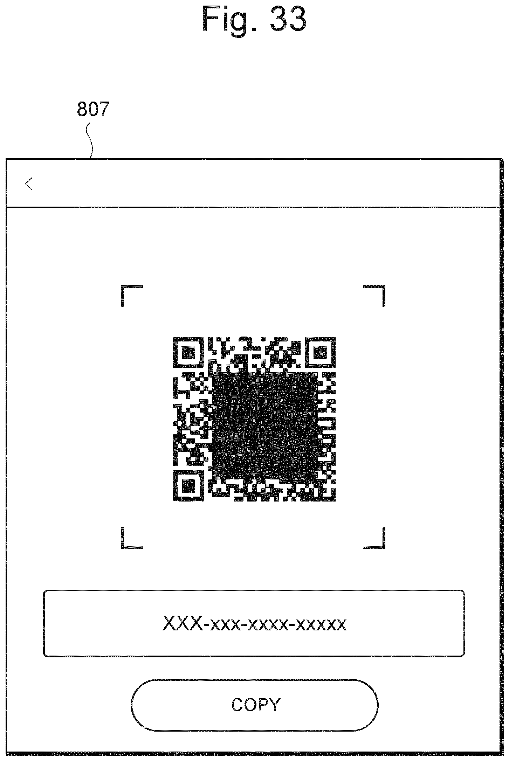

Further, by selecting a button BT8 on the screen 805, a screen 806 to make created challenge information readable on any other terminal as illustrated in Fig. 33 can be generated. Information related to the challenge can be acquired by the other terminal by reading a generated image using the other terminal.

A program described in the first embodiment can be stored on a storage medium. The storage medium with the program stored thereon may be a non-transitory storage medium (non-transitory computer readable medium). Although the non-transitory storage medium is not particularly limited, it may be, for example, a storage medium such as a USB memory or a CD-ROM.

In the first embodiment, the fund provider terminal 10 generates the challenge contract 501, but two or more smart contracts created for challenge assistance may also be created. The smart contracts can be built to perform processes in corporation with the two or more smart contracts to create a challenge with more complex conditions set therefor.

A second embodiment will be described. In the second embodiment and subsequent embodiments, description of matters common to the first embodiment is omitted and only points different from the first embodiment are described.

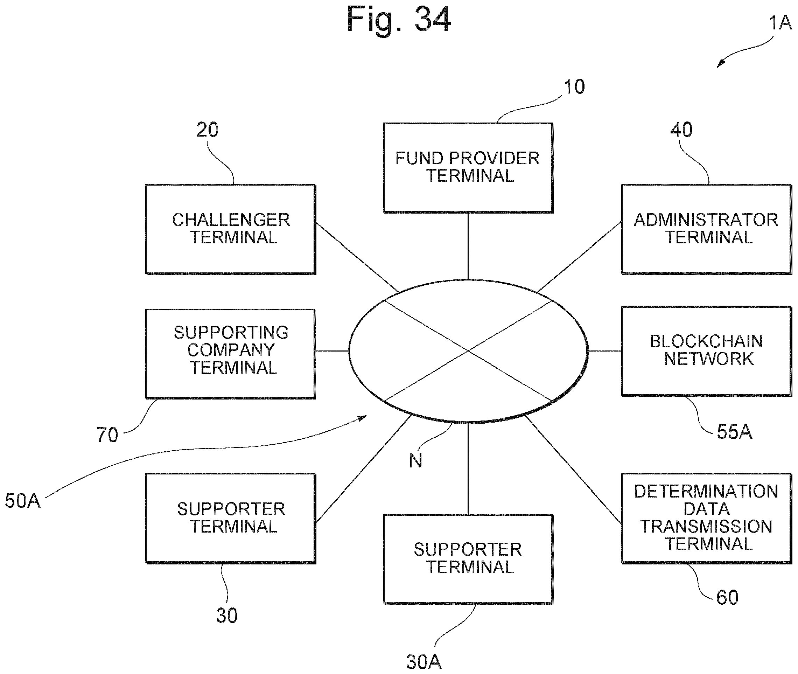

A challenge assistance system 1A according to the second embodiment further includes a supporting company terminal 70 as illustrated in Fig. 34. The supporting company terminal has an account and a public address in a blockchain network 55A.

For example, it is assumed that the supporting company is a beverage maker. A challenger purchases a beverage from the beverage maker as the supporting company. An image indicative of a website address is attached to the beverage. The website of an administrator or the supporting company becomes accessible by reading the image attached to the beverage. On the website, the challenger enters a name, an age, a place of residence, an own public address, a contract address of a challenge contract, a contract address of a thanks token contract, and the like as information about the challenger.

The supporting company sends, to the challenge contract, ETHER, a product exchange token, a cheering token, or the like issued by the supporting company in the blockchain network 55A. The product exchange token or the like sent to the challenge contract may be activated incrementally according to the challenge achievement level.

The product exchange token can be exchanged for a beverage(s) from the supporting company. As the value of the product exchange token, the number of beverages exchangeable for the product exchange token, the expiration date, and the available area can be designed freely. For example, the exchange may not be 1 to 1, or five beverages may be exchanged for one product exchange token.

An example of actual usage of the product exchange token will be described. The product exchange token is managed in a user's wallet. A user goes to a convenience store to read, using an app, a token receiving public address placed next to a cash register and owned by the convenience store to acquire the public address. The user sends the product exchange token to the address. The convenience store checks whether or not the product exchange token received at the token receiving public address own by the convenience store is valid.

The convenience store gives a beverage(s) to the user in exchange for the received product exchange token. The convenience store settles the product exchange token with the administrator or the supporting company later. The convenience store acquires the public address of a user's wallet for the product exchange token by an own terminal not to waste the user's time, and generates a transaction to send the product exchange token from the user to the convenience store. A confirmation screen of the generated transaction is displayed in the app of the user, and when the user approves the content and transmits the product exchange token as a transaction, the product exchange token of the user is passed to the convenience store.

As in the second embodiment, by a dapp configured to combine the product exchange token in addition to the allocation of tokens by the challenge contract 501, the thanks token contract 502, and the cheering token contract 503, the possibility that the user will achieve a challenge can be more increased with the support of a company in addition to the support of family and friends.

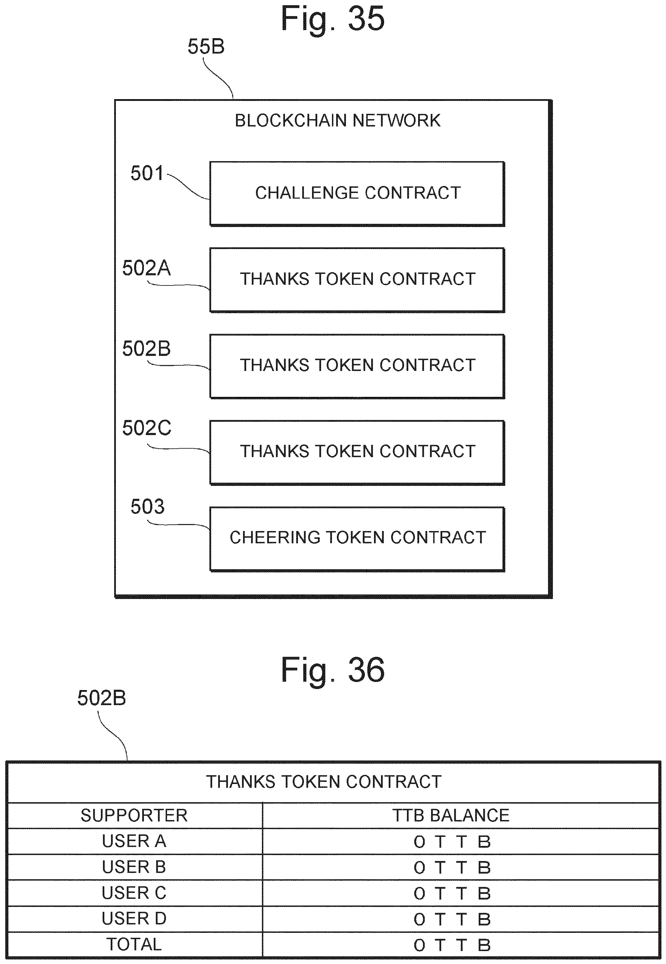

A third embodiment will be described. As illustrated in Fig. 35, a challenge assistance system 1B according to the third embodiment is different in that a thanks token contract 502A, a thanks token contract 502B, and a thanks token contract 502C are provided in a blockchain network 55B.

Like the thanks token contract 502 in the first embodiment, the thanks token contract 502A and the thanks token contract 502C are smart contracts to manage the balances of tokens to be allocated by a challenger for each challenge to supporters for the support of the challenge.

Further, the thanks token contract 502C is related to another challenge separate from the challenge related to the thanks token contract 502A.

The thanks token contract 502B represents the contribution of continuous support to the challenger. In the thanks token contract 502B, the balances are updated based on the thanks token contract 502A. The thanks token contract 502B manages the token balances different from those of the thanks token contract 502A. It is assumed that the token currency units are TTA in the thanks token contract 502A, TTB in the thanks token contract 502B, and TTC in the thanks token contract 502C.

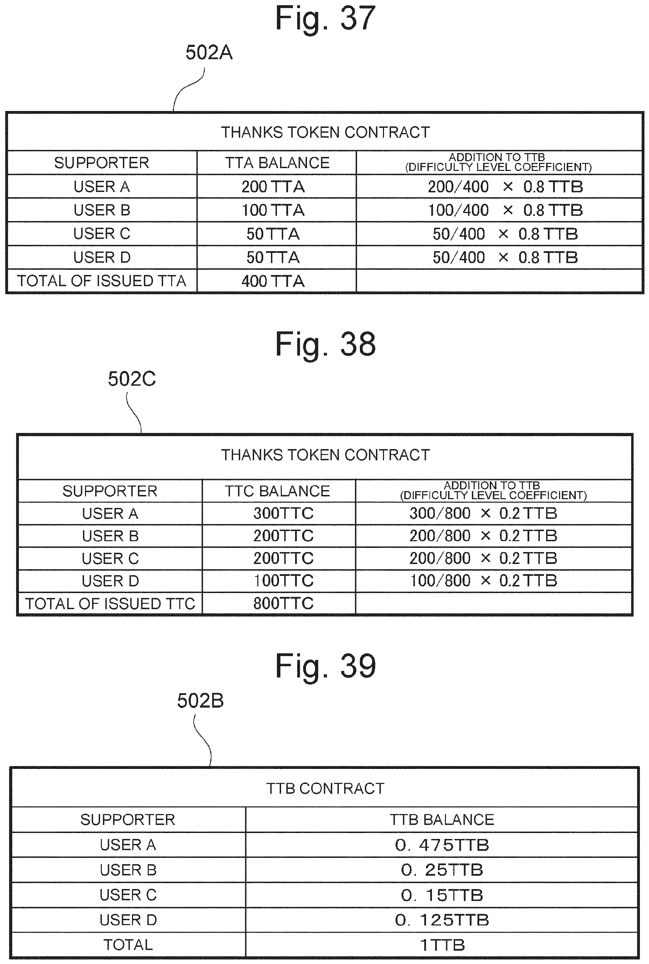

As illustrated in Fig. 36, it is assumed that the initial balances of TTB are all 0 from user A to user D. It is then assumed that the allocation of tokens by the thanks token contract 502A for a certain challenge becomes as illustrated in Fig. 37. The addition amount of each balance of the thanks token contract 502B is determined based on the token balance of the thanks token contract 502A.

The addition amount in the thanks token contract 502B is an amount obtained by multiplying the percentage of issued tokens by a difficulty level coefficient for each user of the thanks token contract 502A. The difficulty level coefficient of the challenge related to the thanks token contract 502A is 0.8.

It is then assumed that the allocation of tokens by the thanks token contract 502C for a certain challenge becomes as illustrated in Fig. 38. Like in the case of the thanks token contract 502A, the addition amount of each balance of the thanks token contract 502B is determined based on the balance of the thanks token contract 502C.

The addition amount in the thanks token contract 502B is an amount obtained by multiplying the percentage of issued tokens by a difficulty level coefficient for each user of the thanks token contract 502C. The difficulty level coefficient of the challenge related to the thanks token contract 502C is 0.2.

The balances of the thanks token contract 502B are updated as illustrated in Fig. 39 by the addition amounts determined based on the allocation of tokens by the thanks token contract 502A and the thanks token contract 502C.

Based on the management of tokens by the thanks token contract 502A or the thanks token contract 502C to manage the contribution of each supporter to a certain challenge alone, the lifetime contribution of the supporter to the challenger is managed like in the thanks token contract 502B, and this enables long-term support.

When a health insurance association, an insurance company, or the like pays incentives to the thanks token contract 502B, rather than to the thanks token contract 502A for temporary supporters, there is a possibility that continuous support will be provided to each member of the association or each insurance subscriber. Further, tokens managed by the thanks token contract 502B may also be designed not to be transferred to any other person.

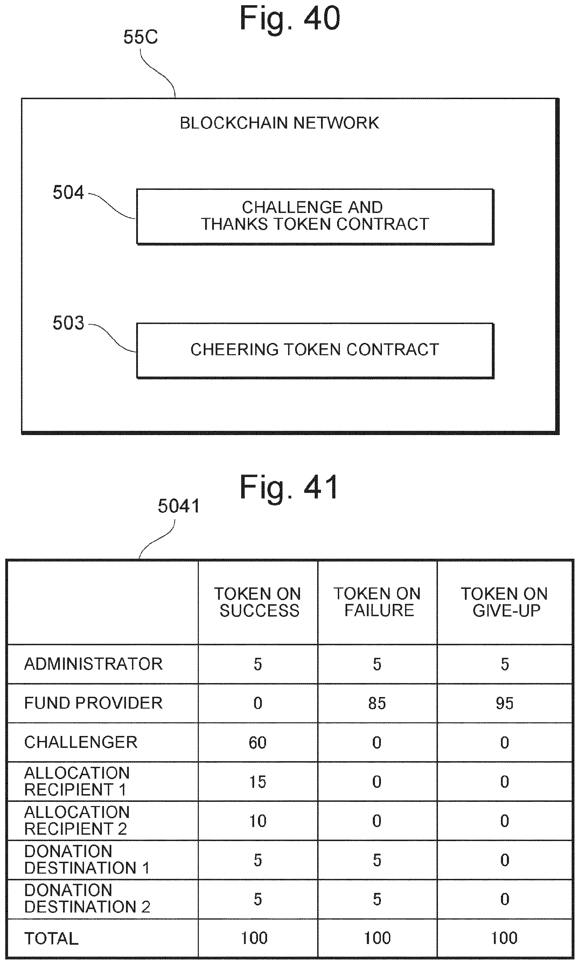

A fourth embodiment will be described. As illustrated in Fig. 40, the fourth embodiment is different from the first embodiment in that a blockchain network 55C has challenge and thanks token contract 504.

The challenge and thanks token contract 504 serves as both the challenge contract 501 and the thanks token contract 502 in the first embodiment. As a token balance 5041 illustrated in Fig. 41, the challenge and thanks token contract 504 allocates three types of tokens corresponding to respective challenge results to each of persons concerned at the start of a challenge, respectively.

It is assumed that each token corresponding to the result after the challenge is determined to be any one of the success, failure, or give-up of the challenge result is valid.

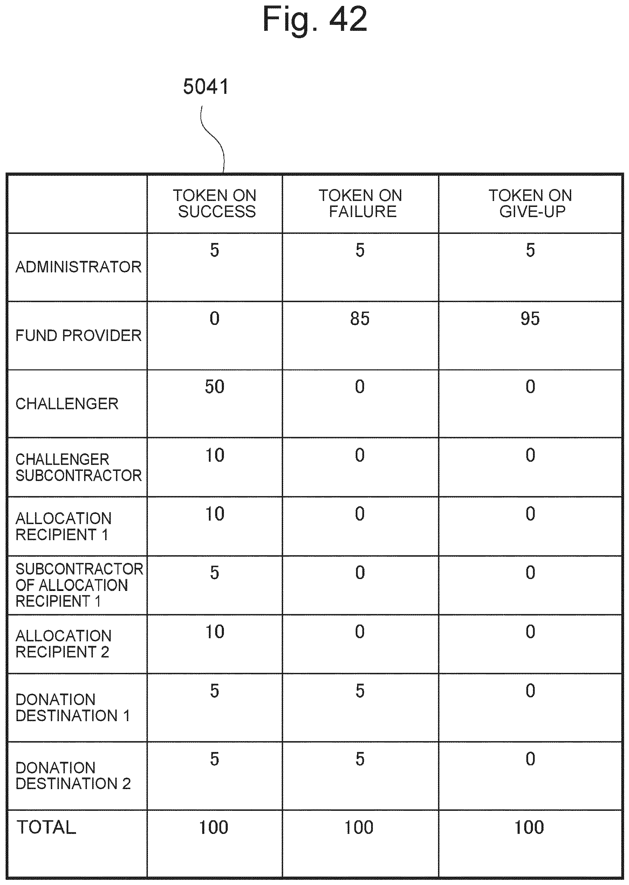

As illustrated in Fig. 42, the challenger can send part of own tokens to a subcontractor to expect own success. Similarly, an allocation recipient can also allocate part of tokens to a subcontractor when the challenge is successful.

If the challenge is successful, since the token at the time of success will be valid, the subcontractor to which the token is allocated supports the challenger more actively.

When the challenge is unsuccessful, the token at the time of success is invalid, and the token at the time of failure is valid. The same applies to the token at the time of give-up.

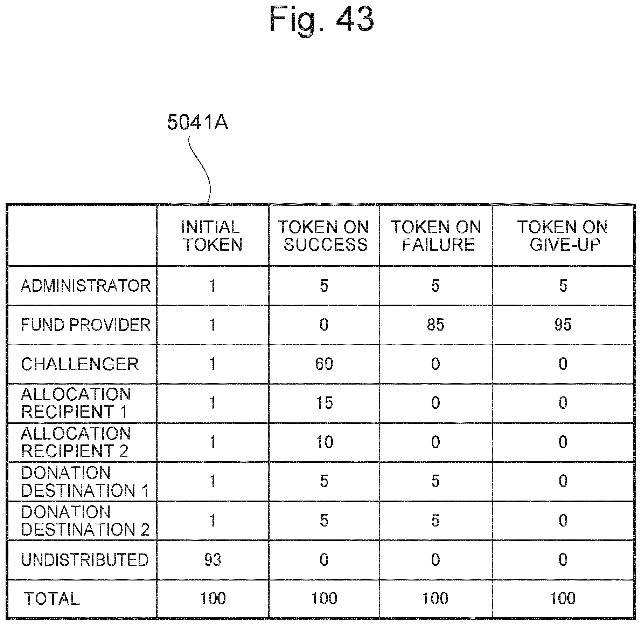

The challenge and thanks token contract 504 may also manage one type of token as a token balance 5041A illustrated in Fig. 43.

When one type of token is managed, a token is distributed to each person concerned as initial allocation at the start of the challenge. The person who received the token can subcontract to get cooperation. For example, it is assumed that the challenger passes 0.2 token to the subcontractor. When the challenge is successful, since the token of the challenger becomes 60 times according to the allocation on success, the subcontractor owns 12 token which is 60 times of 0.2 token.

In the fourth embodiment, tokens are given to the challenger, allocation recipients, and the like during the challenge, and the tokens become valuable according to the result of the challenge. Thus, the challenge can be done while giving a value to each token itself without causing the thanks token to function as an allocation mediator.

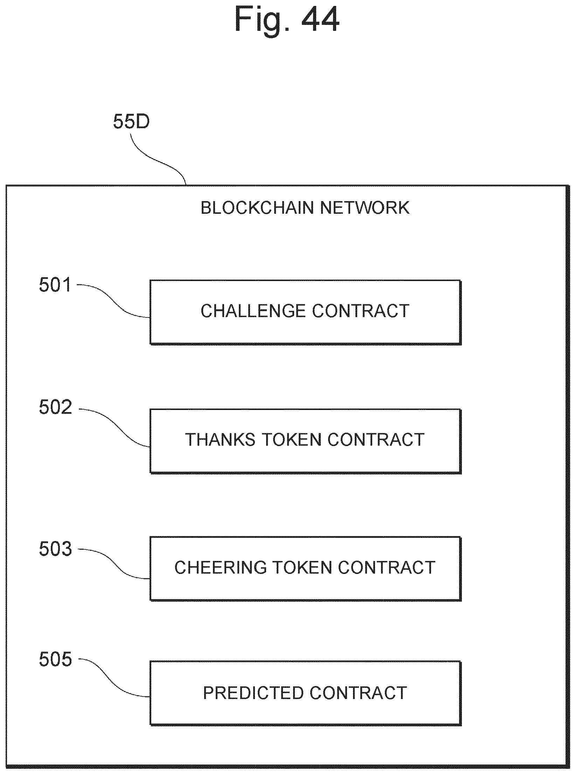

A fifth embodiment will be described. As illustrated in Fig. 44, the fifth embodiment is different from the first embodiment in that a blockchain network 55D has a predicted contract 505. The predicted contract 505 predicts the process of a challenge by the challenger, and success/failure of the challenge to pay a bet with a cheering token.

The predicted contract 505 automatically pays a certain fee to a creator of the predicted contract as soon as the cheering token is received from a supporter or the like. Further, the cheering token is sent to the challenge contract 501. Each of cheering tokens accumulated and held in the challenge contract 501 is allocated to each person concerned under the same conditions as the allocation at the time of success, for example, when the challenge is successful. This cheering token sent to the challenge contract 501 becomes an incentive to do a challenge such as his own walking challenge by taking a risk as depositing his or other people's money.

The embodiments described above are to facilitate the understanding of the present invention, and are not intended to interpret the present invention in a limited manner. Each of elements included in the embodiments, and the arrangement, material, conditions, shape, size, and the like thereof are not limited to those illustrated, and can be changed as appropriate. Further, components illustrated in different embodiments can be partially replaced or combined.

### Reference Signs List

1, 1A, 1B...challenge assistance system, 10...fund provider terminal, 20... challenger terminal, 30, 30A... supporter terminal, 40... administrator terminal, 55, 55A, 55B, 55C, 55D...blockchain network, 60... determination data transmission terminal, 70... supporting company terminal, 80...challenge app, 501 ... challenge contract, 502, 502A, 502B, 502C...thanks token contract, 503... cheering token contract

## Claims

1. A program causing a terminal connected to Internet to execute:
in a blockchain network connected to the Internet and having
a first smart contract which determines whether or not contract terms related to a challenge including conditions to achieve the challenge to be achieved by a first user and a deadline to achieve the challenge are met, and generates a transaction to allocate a first token based on the determination result of the contract terms, and
a second smart contract to which the first token is allocated from the first smart contract, and which manages a balance of a second token allocatable from the first user to a second user and generates a transaction to allocate the first token to the second user based on the balance of the second token of the second user,
a process of acquiring determination data used for the determination of the contract terms;
a process of transmitting the determination data to the first smart contract;
a process of generating a transaction used to allocate the second token from the first user to the second user by the second smart contract; and
a process of transmitting, to the second smart contract, the transaction used to allocate the second token from the first user to the second user.

2. The program according to claim 1, further causing the terminal to execute:
a process of generating token issuance information used to issue the second token; and
a process of transmitting the token issuance information to the blockchain network.

3. The program according to claim 1 or 2, further causing the terminal to execute:
a process of generating a transaction used to allocate the second token from the second user to a third user; and
a process of transmitting, to the second smart contract, the transaction used to allocate the second token from the second user to the third user.

4. The program according to any one of claims 1 to 3, further causing the terminal to execute:
in the blockchain network further having a third smart contract which manages a balance of a third token,
a process of generating a transaction used to allocate the third token by the third smart contract based on the balance of the second token; and
a process of transmitting, to the third smart contract, the transaction used to allocate the third token based on the balance of the second token.

5. The program according to claim 4, further causing the terminal to execute:
a process of generating a transaction used to allocate the third token by the third smart contract based on the balance of the second token and a difficulty level of the challenge; and
a process of transmitting, to the third smart contract, the transaction used to allocate the third token based on the balance of the second token and the difficulty level of the challenge.

6. The program according to any one of claims 1 to 5, further causing the terminal to execute:
a process of generating information indicative of a termination of the challenge; and
a process of transmitting, to the first smart contract, the information indicative of the termination of the challenge to cause the first smart contract to generate a transaction used to allocate the first token according to an allocation setting of the first token when the challenge is terminated.

7. The program according to any one of claims 1 to 6, further causing the terminal to execute:
a process of acquiring a transmission destination to transmit information about the challenge of the first user; and
a process of transmitting, to the transmission destination, the information about the challenge of the first user to generate a transaction used to allocate a prescribed token in the blockchain network from a prescribed user of the blockchain network to the first user.

8. A program causing a terminal connected to Internet to execute:
a process of generating contract terms related to a challenge including conditions to achieve the challenge to be achieved by a first user and a deadline to achieve the challenge, and contract information including an amount of first token deposited for the challenge, an allocation setting of the first token when the challenge is successful, and an allocation setting of the first token when the challenge is unsuccessful; and
a process of determining whether or not the contract terms are met based on the contract terms and determination data used for the determination of the contract terms, and transmitting, to a blockchain network, a contract generation transaction based on the contract information to generate, in the blockchain network connected to the Internet, a first smart contract which generates a transaction to allocate the first token based on the determination result of the contract terms.

9. The program according to claim 8, further causing the terminal to execute:
a process of generating information indicative of a termination of the challenge; and
a process of transmitting, to the first smart contract, the information indicative of the termination of the challenge to cause the first smart contract to generate a transaction to allocate the first token according to an allocation setting of the first token when the challenge is terminated.

10. The program according to any one of claims 1 to 9, wherein the first smart contract calculates an achievement level of the challenge based on the contract terms and the determination data, and generates a transaction to allocate the first token based on the calculated achievement level.

11. The program according to any one of claims 1 to 10, wherein the determination data includes end determination data to determine end of the challenge.

12. The program according to claim 11, wherein the end determination data includes data about date and time.

13. A program causing a terminal connected to Internet to execute:
a process of determining whether or not contract terms related to a challenge including conditions to achieve the challenge to be achieved by a first user and a deadline to achieve the challenge are met, and generating a transaction to allocate a first token based on the determination result of the contract terms; and transmitting, to a blockchain network, a contract generation transaction to generate, in the blockchain network, a smart contract which manages a balance of a second token allocatable from the first user to a second user and activated after the determination is made, and generates a transaction to allocate the second token to the second user based on the determination result of the contract terms.

14. A program causing a terminal connected to Internet to execute:
in a blockchain network having
a first smart contract which determines whether or not contract terms related to a challenge including conditions to achieve the challenge to be achieved by a prescribed user and a deadline to achieve the challenge are met based on the contract terms and determination data used for the determination of the contract terms, and
a second smart contract in which a token from the blockchain network connected to the Internet is accumulated and which allocates the accumulated token based on the determination result transmitted from the first smart contract,
a process of generating a transaction to bet the token on a prediction of the determination result; and
a process of transmitting, to the second smart contract, the transaction to bet the token on the prediction of the determination result.

15. A challenge assistance system comprising
a first terminal and a second terminal connected to Internet, wherein
the first terminal includes:
a contract information generation unit which generates contract terms related to a challenge including conditions to achieve the challenge to be achieved by a user and a deadline to achieve the challenge, and contract information including an amount of first token deposited for the challenge, an allocation setting of the first token when the challenge is successful, and an allocation setting of the first token when the challenge is unsuccessful; and
a first transaction generation unit which determines whether or not the contract terms are met based on the contract terms and determination data used for the determination of the contract terms, and transmits, to a blockchain network, a contract generation transaction based on the contract information to generate, in the blockchain network connected to the Internet, a smart contract which generates a transaction to allocate the first token based on the determination result of the contract terms, and
the second terminal includes:
a determination data acquisition unit which acquires the determination data; and
a second transaction generation unit which transmits the determination data to the smart contract.

16. The challenge assistance system according to claim 15, wherein
the user is a first user,
the smart contract is a first smart contract,
the blockchain network has a second smart contract to which the first token is allocated from the first smart contract, and which manages a balance of a second token allocatable from the first user to a second user, and generates a transaction to allocate the first token to the second user based on the balance of the second token of the second user,
the second transaction generation unit generates a transaction used to allocate the second token by the second smart contract from the first user to the second user, and
the second transaction generation unit transmits, to the second smart contract, the transaction used to allocate the second token from the first user to the second user.

17. A challenge assistance method, comprising:
a step of causing a first terminal connected to Internet to generate contract terms related to a challenge including conditions to achieve the challenge to be achieved by a user and a deadline to achieve the challenge, and contract information including an amount of first token deposited for the challenge, an allocation setting of the first token when the challenge is successful, and an allocation setting of the first token when the challenge is unsuccessful;
a step of causing the first terminal to determine whether or not the contract terms are met based on the contract terms and determination data used for the determination of the contract terms, and to transmit, to a blockchain network, a contract generation transaction based on the contract information to generate, in the blockchain network connected to the Internet, a smart contract which generates a transaction to allocate the first token based on the determination result of the contract terms;
a step of causing a second terminal connected to the Internet to acquire the determination data; and
a step of causing the second terminal to transmit the determination data to the smart contract.

18. The challenge assistance method according to claim 17, wherein
the user is a first user,
the smart contract is a first smart contract, and
the blockchain network has a second smart contract to which the first token is allocated from the first smart contract, and which manages a balance of a second token allocatable from the first user to a second user, and generates a transaction to allocate the first token to the second user based on the balance of the second token of the second user, the challenge assistance method further comprising:
a step of causing the first terminal to generate a transaction used to allocate the second token by the second smart contract from the first user to the second user; and
a step of causing the first terminal to transmit, to the second smart contract, the transaction used to allocate the second token from the first user to the second user.

19. A terminal comprising:
a determination data acquisition unit which acquires determination data used to determine contract terms related to a challenge including conditions to achieve the challenge to be achieved by a user and a deadline to achieve the challenge; and
a transaction generation unit provided in a blockchain network to transmit the determination data to a smart contract which determines whether or not the contract terms are met and generates a transaction to allocate a first token based on the determination result of the contract terms.

20. The terminal according to claim 19, wherein
the user is a first user,
the smart contract is a first smart contract,
the blockchain network has a second smart contract to which the first token is allocated from the first smart contract, and which manages a balance of a second token allocatable from the first user to a second user, and generates a transaction to allocate the first token to the second user based on the balance of the second token of the second user,
the transaction generation unit generates a transaction used to allocate the second token by the second smart contract from the first user to the second user, and
the transaction generation unit transmits, to the second smart contract, the transaction used to allocate the second token from the first user to the second user.

21. A terminal comprising:
a contract information generation unit which generates contract terms related to a challenge including conditions to achieve the challenge to be achieved by a first user and a deadline to achieve the challenge, and contract information including an amount of first token deposited for the challenge, an allocation setting of the first token when the challenge is successful, and an allocation setting of the first token when the challenge is unsuccessful; and
a transaction generation unit which determines whether or not the contract terms are met based on the contract terms and determination data used for the determination of the contract terms, and transmits, to a blockchain network, a contract generation transaction based on the contract information to generate, in the blockchain network, a smart contract which generates a transaction to allocate the first token based on the determination result of the contract terms.

22. A program causing a terminal connected to Internet to execute
a process of determining whether or not contract terms related to a challenge including conditions to achieve the challenge to be achieved by a user and a deadline to achieve the challenge are met based on the contract terms and determination data used for the determination of the contract terms, and transmitting contract information to a blockchain network to generate, in the blockchain network, a smart contract which generates a transaction to allocate a first token based on the determination result of the contract terms.

23. A program causing a terminal connected to Internet to execute
a process of transmitting contract information to a blockchain network to generate, in the blockchain network, a smart contract to which a first token is allocated, and which manages a balance of a second token allocatable from a first user to a second user and generates a transaction to allocate the first token to the second user based on the balance of the second token of the second user.
